(19) Europäisches Patentamt
European Patent Office
Office européen des brevets

(11) EP 0 955 286 B1

(12) # FASCICULE DE BREVET EUROPEEN

(45) Date de publication et mention de la délivrance du brevet:
**07.08.2002 Bulletin 2002/32**

(21) Numéro de dépôt: **99401091.6**

(22) Date de dépôt: **05.05.1999**

(51) Int Cl.[7]: **C07C 59/54**, C07C 59/56, C07C 59/72, C07C 69/732, C07C 69/734, C07C 235/34, C07D 311/58, A61K 31/19, A61K 31/215, A61K 31/16, A61K 31/35, C07C 47/57, C07C 47/575

(54) **Dérivés du dihydro ou tétrahydronaphtalène ayant une activité (anti-)oestrogène**

Dihydro- oder Tetrahydronaphthalenderivate mit (anti)estrogenen Eigenschaften

Dihydro- or tetrahydronaphthalene derivatives having (anti-)estrogen activity

(84) Etats contractants désignés:
**AT BE CH CY DE DK ES FI FR GB GR IE IT LI LU NL PT SE**

(30) Priorité: **06.05.1998 FR 9805709**

(43) Date de publication de la demande:
**10.11.1999 Bulletin 1999/45**

(73) Titulaire: **Aventis Pharma S.A.**
**92160 Antony (FR)**

(72) Inventeur: **Nique, Francois**
**94170 Le Perreux sur Marne (FR)**

(56) Documents cités:
**WO-A-97/32837** **US-A- 5 552 412**

- **WILLSON TM ET AL: "3-[4-(1,2-Diphenylbut-1-eny)phenyl] acrylic acid: a non-steroidal estrogen with functional selectivity for bone over uterus in rats" JOURNAL OF MEDICINAL CHEMISTRY, vol. 37, no. 11, 27 mai 1994 (1994-05-27), pages 1550-3, XP002091552**

Remarques:
Le dossier contient des informations techniques présentées postérieurement au dépôt de la demande et ne figurant pas dans le présent fascicule.

EP 0 955 286 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention a pour objet des dérivés du dihydro ou tétrahydronaphtalène, leurs procédés et intermédiaires de préparation, leur application thérapeutique et les compositions pharmaceutiques les renfermant.

L'ostéoporose est une pathologie qui se caractérise par une réduction quantitative et qualitative du tissu osseux, suffisante pour entraîner des fractures vertébrales ou périphériques, ce de façon spontanée ou à l'occasion de traumatisme minime. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post ménopausique peut être compensée par une hormonothérapie substitutive où l'oestrogène joue un rôle majeur en préservant le capital osseux. Mais l'estrogénothérapie au long cours s'accompagne parfois d'effet indésirable sur l'appareil génital (hyperplasie endométriale, tumeur mammaire...), ce qui constitue un inconvénient majeur et limite son application.

Il convient donc de trouver d'autres composés que l'oestradiol ayant une activité oestrogène dissociée, à savoir une activité oestrogène au niveau osseux, tout en n'ayant pas ou peu d'activité d'hyperplasie endométriale, ni d'activité de prolifération de tumeur mammaire.

L'invention a donc pour objet les composés de formule générale (I) :

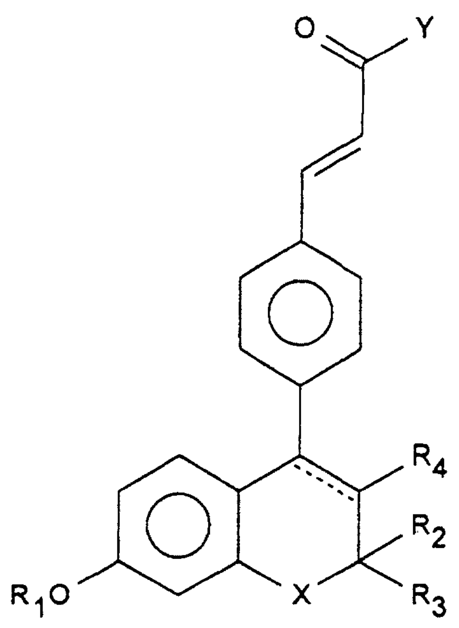

(I)

dans laquelle :

- $R_1$ représente un atome d'hydrogène, un radical alkyle ou un radical acyle,
- $R_2$ et $R_3$, identiques ou différents, représentent un atome d'hydrogène, un radical alkyle, ou forment ensemble un cyclo alkyle renfermant de 3 à 7 atomes de carbone,
- $R_4$ représente un radical aryle ou hétéroaryle,
- X représente O ou $CH_2$,
- Y représente un radical hydroxy, alkyloxy ou un groupement NRaRb dans lequel :
  - soit Ra et Rb, identiques ou différents, représentent un atome d'hydrogène ou un radical alkyle,
  - soit Ra et Rb forment ensemble avec l'atome d'azote auquel ils sont liés un hétérocycle mono ou polycyclique de 3 à 15 chaînons renfermant éventuellement un hétéroatome additionnel choisi parmi l'oxygène, le soufre et l'azote, lesdits alkyle, alkyloxy et acyle renferment de 1 à 6 atomes de carbone, lesdits alkyle, acyle, aryle et hétérocycle sont substitués ou non substitués, le trait en pointillé représente éventuellement une double liaison,

les composés de formule (I) pouvant être sous toutes leurs formes stéréoisomères possibles, isolés ou en mélanges, ainsi que les sels d'addition avec les acides ou les bases.

Par alkyle renfermant de 1 à 6 atomes de carbone, on désigne les radicaux méthyle, éthyle, propyle, isopropyle, butyle, isobutyle, tert-butyle, n-pentyle, n-hexyle, 2-méthyl pentyle, 2,3-diméthylbutyle.

Par acyle renfermant de 2 à 6 atomes de carbone on désigne notamment les radicaux acétyle, propionyle et butyryle.

Par radical aryle on entend de préférence le groupement phényle ou naphtyle.

Par radical hétéroaryle (ou hétérocycle aromatique), on entend de préférence les groupements thiényle, furyle, isothiényle, isofuryle, thiazolyle, isothiazolyle, oxazolyle, thiadiazolyle, pyridinyle.

Par radical alkyloxy renfermant de 1 à 6 atomes de carbone, on entend de préférence les radicaux méthoxy, éthoxy, propyloxy, isopropyloxy et butyloxy.

Lorsque Ra et Rb forment ensemble avec l'atome d'azote auquel ils sont liés un hétérocycle saturé, il s'agit notamment des hétérocycles mono ou bicycliques suivants :

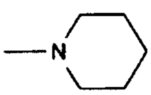 , 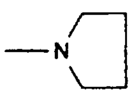 , 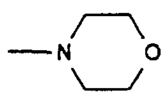 , 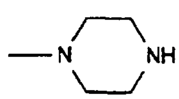 et 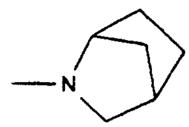

Lorsque les radicaux alkyle, acyle, aryle ou hétérocycle (aromatique ou non) sont substitués, ils peuvent l'être notamment par les radicaux suivants :

- halogène, à savoir fluor, chlore, brome ou iode, alkoxy tel que méthoxy, éthoxy, propyloxy, isopropyloxy, butyloxy, alkylthio tel que méthylthio, éthylthio, propylthio, isopropylthio, butylthio, amino, alkylamino tel que méthylamino ou éthylamino, dialkylamino tel que diméthylamino, diéthylamino, méthyléthylamino, chacun de ces radicaux dialkylamino étant éventuellement sous forme oxydée, aminoalkyle tel que aminométhyle ou aminoéthyle, dialkylaminoalkyle tel que diméthylamino méthyle ou éthyle, dialkylaminoalkyloxy tel que diméthylamino éthyloxy, hydroxyle éventuellement acylé, acyle tel que acétyle, propionyle, butyryle, benzoyle, carboxy libre, estérifié tel que alkoxy carbonyle par exemple méthoxy carbonyle ou éthoxy carbonyle, cyano, trifluorométhyle, aryle tel que phényle, aralkyle tel que benzyle, alkyle, alkényle ou alkynyle ces radicaux étant eux-mêmes éventuellement substitués par les radicaux halogène, alkyle, alkoxy, alkylthio, amino, alkylamino ou dialkylamino indiqués ci-dessus.

Bien entendu, l'expression "substitué" indique qu'un ou plusieurs substituants, identiques ou différents, peuvent être présents. A titre d'exemple, lorsque le groupement alkyl est un radical méthyl substitué par un ou plusieurs atomes d'halogène, il peut s'agir notamment de $CH_2Cl$, $CH_2F$, $CHF_2$ et $CF_3$.

L'invention s'étend naturellement aux sels des composés de formule (I), comme par exemple les sels formés avec des acides minéraux ou organiques sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques. Lorsque les composés de formule (I) comportent une fonction acide, l'invention s'étend aux sels des métaux alcalins, alcalino terreux ou d'ammonium éventuellement substitués.

L'invention a plus particulièrement pour objet les composés de formule (I) telle que définie plus haut dans laquelle :

$R_1$, $R_2$ et $R_3$ sont des atomes d'hydrogène, $R_4$ est un phényle substitué ou non substitué et Y représente un radical hydroxy, alkyloxy renfermant de 1 à 6 atomes de carbone ou NRaRb, Ra et Rb représentant chacun un radical alkyle renfermant de 1 à 6 atomes de carbone.

L'invention a également plus particulièrement pour objet les composés de formule (I) telle que définie précédemment dans laquelle les groupements $R_4$ et Ph-CH=CH-COY sont en position cis.

L'invention a tout particulièrement pour objet les composés suivants :

- acide 3-[4-(3,4-dihydro-6-hydroxy-2-phényl-1-naphtalényl) phényl] 2-propénoïque,
- (+,-) cis acide 3-[4-(6-hydroxy-2-phényl-1,2,3,4-tétrahydro-1-naphtalényl)phényl] 2-propénoïque,

- (+,-) cis N,N-diéthyl-3-[4-(6-hydroxy-2-phényl-1,2,3,4-tétrahydro-1-naphtalényl)phényl] 2-propénamide,
- 3-[4-(3,4-dihydro-6-méthoxy-2-(4-méthoxyphényl)-naphtalényl) phényl] 2-propénoate d'éthyle,
- 3-[4-(3,4-dihydro-6-hydroxy-2-(4-hydroxyphényl) 1-naphtalényl) phényl] 2-propénoate d'éthyle,
- acide 3-[4-(3,4-dihydro-6-hydroxy-2-(4-hydroxyphényl)-naphtalényl) phényl] 2-propénoïque,
- acide 3-[4-(6-méthoxy-2-(4-méthoxyphényl) 3,4-dihydro 1-naphtalényl) phényl] 2-propénoïque,
- N,N-diéthyl 3-[4-[3,4-dihydro-6-méthoxy-2-(4-méthoxyphényl) 1-naphtalényl] phényl] 2-propénamide,
- N,N-diéthyl 3-[4-[3,4-dihydro-6-hydroxy-2-(4-hydroxyphényl) 1-naphtalényl] phényl] 2-propénamide,
- (+,-) cis 3-[4-[6-méthoxy-2-(4-méthoxyphényl) 1,2,3,4-tétrahydro 1-naphtalényl) phényl] 2-propénoate d'éthyle,
- (+,-) cis 3-[4-[6-hydroxy-2-(4-hydroxyphényl) 1,2,3,4-tétrahydro 1-naphtalényl] phényl] 2-propénoate d'éthyle,
- (+,-) cis acide 3-[4-[3,4-[6-méthoxy-2-(4-méthoxyphényl)] 1,2,3,4-tétrahydro-1-naphtalényl] phényl] 2-propénoîque,
- (+,-) cis acide 3-[4-[6-hydroxy-2-(4-hydroxyphényl) 1,2,3,4-tétrahydro 1-naphtalényl] phényl] 2-propénoïque,
- (+,-) cis N,N-diéthyl-3-[4-[6-méthoxy-2-(4-méthoxyphényl) 1,2,3,4-tétrahydro 1-naphtalényl] phényl] 2-propénamide,
- (+,-) cis N,N-diéthyl-3-[4-[6-hydroxy-2-(4-hydroxyphényl) 1,2,3,4-tétrahydro 1-naphtalényl] phényl] 2-propénamide,
- 3-[4-[3,4-dihydro-6-méthoxy-2-(4-fluorophényl) 1-naphtalényl) phényl] 2-propénoate d'éthyle,
- 3-[4-[3,4-dihydro-6-hydroxy-2-(4-fluorophényl) 1-naphtalényl) phényl] 2-propénoate d'éthyle,
- acide 3-[4-[3,4-dihydro-6-hydroxy-2-(4-fluorophényl) 1-naphtalényl) phényl] 2-propénoïque,
- acide 3-[4-[6-méthoxy-2-(4-fluorophényl) 3,4-dihydro 1-naphtalényl] phényl] 2-propénoïque,
- N,N-diéthyl 3-[4-[3,4-dihydro-6-méthoxy-2-(4-fluorophényl) 1-naphtalényl] phényl] 2-propénamide,
- N,N-diéthyl 3-[4-[3,4-dihydro-6-hydroxy-2-(4-fluorophényl) 1-naphtalényl] phényl] 2-propénamide,
- (+,-) cis 3-[4-[6-méthoxy-2-(4-fluorophényl) 1,2,3,4-tétrahydro 1-naphtalényl] phényl] 2-propénoate d'éthyle,
- (+,-) cis 3-[4-[6-hydroxy-2-(4-fluorophényl) 1,2,3,4-tétrahydro 1-naphtalényl] phényl] 2-propénoate d'éthyle,
- (+,-) cis acide 3-[4-[2-(4-fluorophényl) 6-hydroxy 1,2,3,4-tétrahydro 1-naphtalényl] phényl] 2-propénoïque,
- (+,-) cis acide 3-[4-[2-(4-fluorophényl) 6-méthoxy 1,2,3,4-tétrahydro 1-naphtalényl] phényl] 2-propénoïque,
- (+,-) cis N,N-diéthyl 3-[4-[6-méthoxy-2-(4-fluorophényl) 1,2,3,4-tétrahydro 1-naphtalényl] phényl] 2-propénamide,
- (+,-) cis N,N-diéthyl 3-[4-[6-hydroxy-2-(4-fluorophényl) 1,2,3,4-tétrahydro 1-naphtalényl] phényl] 2-propénamide,
- 3-[4-(3,4-dihydro-6-méthoxy-2-phényl-1-naphtalényl) phényl] 2-propénoate d'éthyle,
- 3-[4-(3,4-dihydro-6-hydroxy-2-phényl-1-naphtalényl) phényl] 2-propénoate d'éthyle,
- (+,-) cis 3-[4-(6-méthoxy-2-phényl) 1,2,3,4-tétrahydro-1-naphtalényl) phényl] 2-propénoate d'éthyle,
- (+,-) cis 3-[4-(6-hydroxy-2-phényl-1,2,3,4-tétrahydro-1-naphtalényl) phényl] 2-propénoate d'éthyle,
- (+,-) cis N,N-diéthyl 3-[4-(6-méthoxy-2-phényl-1,2,3,4-tétrahydro 1-naphtalényl) phényl] 2-propénamide,

ces composés de formule (I) pouvant être sous toutes leurs formes stéréoisomères possibles isolées ou en mélange ainsi que les sels d'addition avec les acides et les bases.

L'invention a également pour objet un procédé de préparation des composés de formule (I) telle que définie précédemment, caractérisé en ce que l'on soumet un composé de formule (II) :

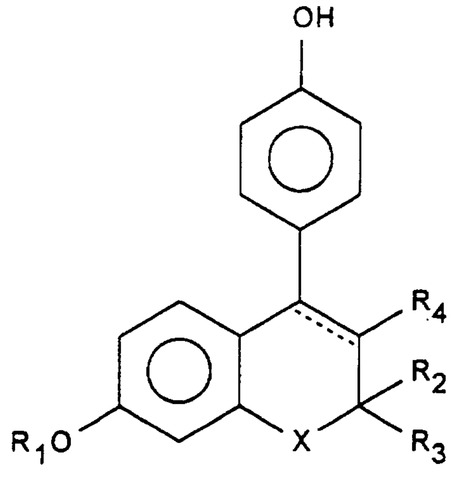

(II)

à l'action d'un agent activant du phénol, puis à l'action d'un composé de formule :

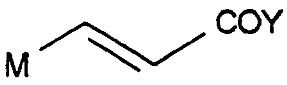

(III)

dans laquelle M est un dérivé organométallique, de préférence organostannique, et Y est tel que défini précédemment, afin d'obtenir le composé de formule (I) qui le cas échéant, si désiré ou si nécessaire et dans un ordre approprié est mis en oeuvre dans l'une ou plusieurs des réactions suivantes :

- alkylation/déalkylation en position 6,
- acylation/clivage de l'acyloxy en position 6,
- estérification/saponification de l'acide ou de l'ester que représente COY,
- amidification de l'acide ou de l'ester que représente COY,
- salification,
- séparation des diastéréoisomères, résolution et/ou transformation en un autre isomère.

Par agent activant du phénol, on entend notamment un agent permettant la formation du triflate ou du mésylate, par action de l'anhydride correspondant en milieu basique.
L'action du composé de formule (III) sur le phénol (II) activé s'effectue selon les conditions décrites par M.R. PEÑA et J.K. STILLE, J. Am. Chem. Soc., 111, (1989), 5417-5424.
Le composé de formule (III) est de préférence un stannane et la réaction s'effectue en présence d'un catalyseur tel que le tétrakis triphényl phosphine palladium.
Les réactions d'alkylation, de déalkylation en position 3, d'acylation, de clivage de l'acyloxy en position 3, d'estérification, de saponification, d'acidification et de salification s'effectuent selon les méthodes connues de l'homme du métier.
L'invention a également pour objet un procédé de préparation des composés de formule (I) dans laquelle le trait en pointillé représente une double liaison, caractérisé en ce que l'on soumet un composé de formule (IV) :

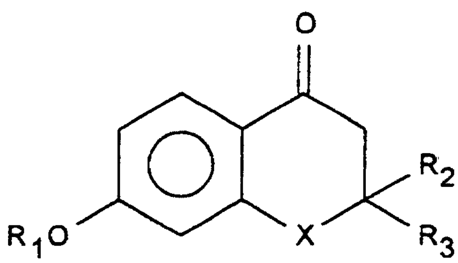

(IV)

$R_1$, $R_2$, $R_3$ et X étant tels que définis précédemment à l'action d'un composé organo-métallique, notamment magnésium ou lithium, dérivé du 4-halogéno benzaldéhyde protégé, de préférence en présence de chlorure de cérium, afin d'obtenir, après traitement acide, le composé de formule (V) :

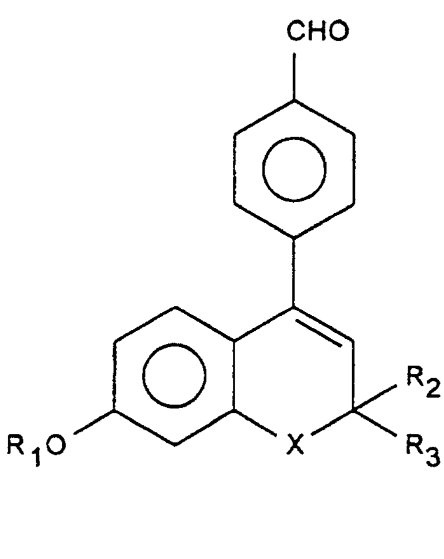

(V)

composé de formule (V) que l'on soumet à l'action d'un réactif d'halogénation afin d'obtenir un composé de formule (VI) :

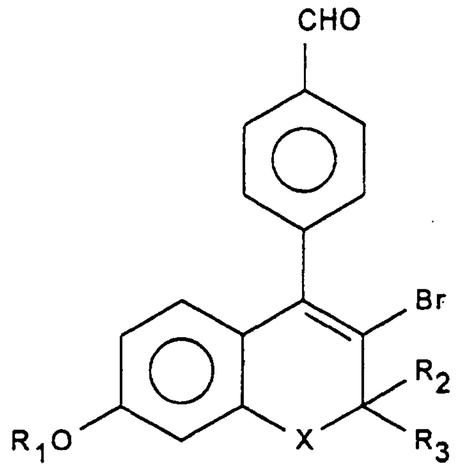

(VI)

composé de formule (VI) que l'on soumet à l'action d'un réactif d'arylation, afin d'obtenir un composé de formule (VII) :

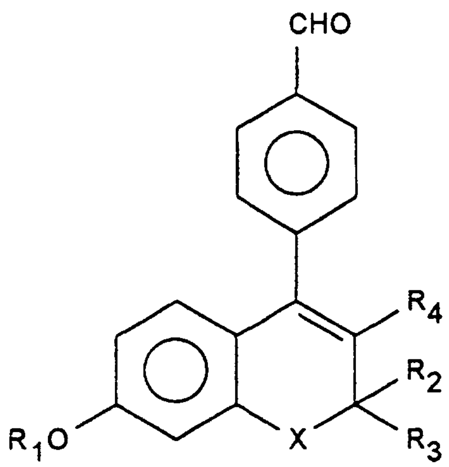

(VII)

composé de formule (VII) que l'on soumet à l'action d'un réactif de wittig de formule :

$$\phi_3P=CH-COY$$

dans laquelle Y est tel que défini précédemment afin d'obtenir le composé de formule (I) qui, le cas échéant, si désiré et si nécessaire et dans un ordre approprié est mis en oeuvre dans l'une ou plusieurs des réactions suivantes :

- alkylation/déalkylation en position 6,
- acylation/clivage de l'acyloxy en position 6,
- estérification/saponification de l'acide ou de l'ester que représente COY,
- amidification de l'acide ou de l'ester que représente COY,
- salification,
- séparation des diastéréoisomères, résolution et/ou transformation en un autre isomère.

L'action du 4-halogéno benzaldéhyde protégé, sur le composé de formule (IV) s'effectue selon les conditions décrites par T. IMAMOTO et coll., J. Am. Chem. Soc., 111, (1989), 4392.

L'introduction d'un halogène, notamment un brome en position 2 afin d'obtenir le composé de formule (VI) s'effectue selon la méthode décrite dans le brevet WO 96/21656. Une méthode préférée est l'action du perbromure

de pyridinium dans le tétrahydrofuranne.

La réaction de substitution d'un halogène vinylique par un aryle ou un hétéroaryle afin d'obtenir le composé de formule (VII) s'effectue selon la méthode décrite par T.M. WILSON et Coll., J. Med. Chem., 37, (1994), 1550.

Une méthode préférée pour l'introduction d'un phényle consiste en l'action d'acide phénylboronique en présence de palladium tétrakis triphényl phosphine et de bicarbonate de sodium en milieu tétrahydrofuranne/eau.

La réaction de Wittig s'effectue selon les méthodes classiques connues de l'homme du métier. On utilise de préférence un réactif dans lequel Y est un groupement alkyloxy tel que le carboéthoxyméthylènetriphénylphosphorane.

Les composés de formule générale (I) ainsi que leurs sels d'addition avec les acides pharmaceutiquement acceptables possèdent notamment des activités oestrogène, anti-oestrogène et antiproliférative.

A ce titre, les composés de formule (I) peuvent être utilisés dans le traitement des troubles liés à une hypofolliculinie, par exemple les aménorrhées, les dysménorrhées, les avortements répétés, les troubles prémenstruels, dans le traitement de certaines pathologies estrogèno-dépendantes telles que les adénomes ou les carcinomes prostatiques, les carcinomes mammaires et ses métastases ou le traitement des tumeurs bénignes du sein, en tant qu'anti-utérotrophique ainsi que dans le traitement substitutif de la ménopause ou de la périménopause.

Parmi les symptômes et les conséquences liées à la ménopause, on entend plus précisément les bouffées de chaleur, les sueurs, l'atrophie et la sécheresse vaginale, les symptômes urinaires et à long terme la diminution de la masse osseuse et l'augmentation du risque de fracture, ainsi que la perte de la protection cardio-vasculaire offerte par les oestrogènes.

En particulier, les composés de formule (I) ainsi que leurs sels d'addition avec les acides ou les bases pharmaceutiquement acceptables, peuvent ainsi être utilisés dans la prévention ou le traitement de l'ostéoporose.

Les composés de formule (I) ainsi que leurs sels d'addition avec les acides ou les bases pharmaceutiquement acceptables, peuvent également être utilisés dans la prévention ou le traitement de l'ostéoporose chez l'homme.

Ils peuvent également être utilisés dans la prévention ou le traitement des ostéoporoses secondaires (par exemple cortisoniques ou liées à une immobilisation).

Les composés de formule (I) ainsi que leurs sels d'addition avec les acides ou les bases pharmaceutiquement acceptables possèdent notamment une activité estrogénique dissociée.

Par activité estrogénique dissociée, on entend une activité estrogénique au niveau osseux tout en ne manifestant qu'une activité minimale au niveau utérin entraînant ainsi l'absence de prolifération endométriale (activité bien inférieure à celle de l'oestradiol).

Par ailleurs, les composés selon l'invention présentent les avantages suivants :

- Ils présentent une activité anti-oestrogène et/ou antiproliférative au niveau du sein. A l'opposé de l'oestradiol ils ne stimulent pas la croissance de cellules tumorales mammaires humaines et même peuvent inhiber leur croissance. Les composés selon l'invention sont donc particulièrement avantageux pour le traitement de la ménopause en ce qui concerne les femmes à risque de cancer mammaire (antécédents familiaux) qui sont donc exclues d'un traitement substitutif par l'oestradiol.

Ils peuvent être également utilisables dans le traitement des cancers mammaires.

- Ils entraînent un abaissement du taux de cholestérol sérique à un niveau au moins équivalent à celui induit par l'estradiol. Ils renforcent ainsi la protection cardiovasculaire.
- Enfin les composés selon l'invention ne présentant aucune activité oestrogène au niveau utérin, ne nécessitent pas d'être administrés en association avec un composé progestomimétique.

L'invention a donc pour objet les composés de formule (I) ainsi que leurs sels d'addition avec les acides ou les bases pharmaceutiquement acceptables, à titre de médicaments.

L'invention a plus particulièrement pour objet les composés de formule (I) telle que définie précédemment ainsi que leurs sels d'addition avec les acides ou les bases pharmaceutiquement acceptables, à titre de médicaments destinés à la prévention ou au traitement de l'ostéoporose.

L'invention s'étend aux compositions pharmaceutiques renfermant comme principe actif au moins l'un des médicaments tels que définis ci-dessus ainsi qu'un ou plusieurs excipients pharmaceutiquement acceptables.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, d'anneaux intravaginal, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques,

les glycols, les divers agents mouillants, dispersants ou émulsifiants, les conservateurs.

La posologie utile varie en fonction de l'affection à traiter et de la voie d'administration ; elle peut varier par exemple de 1 à 1000 mg par jour chez l'adulte par voie orale.

Les composés de formule (II) avec X = $CH_2$ sont décrits dans le brevet US 3947520. Les composés de formule (II) avec X = O sont aisément accessibles par la méthode décrite dans ce brevet.

Les composés de formule (IV) sont pour la plupart commerciaux ou aisément accessibles par l'homme du métier.

L'invention a également pour objet à titre de produits intermédiaires, les composés de formules (V), (VI) et (VII) tels que définis précédemment.

Les exemples ci-dessous illustrent l'invention sans toutefois la limiter.

Solvants décrits dans les exemples : AcOEt (acétate d'éthyle), TEA (triéthylamine), $CH_2Cl_2$ (dichlorométhane), $CHCl_3$ (chloroforme), MeOH (méthanol), $NH_4OH$ (hydroxyde d'ammonium), iPrOH (alcool isopropylique), EP (éther de pétrole).

**EXEMPLE 1 : 3-[4-(3,4-dihydro-6-méthoxy-2-phényl-1-naphtalényl)phényl]-2-propénoate d'éthyle**

Stade A : Introduction du benzaldéhyde en position 1 : 4-(3,4-dihydro-6-méthoxy-1-naphtalényl)-benzaldéhyde

**1) Déshydratation de $CeCl_3$**

On chauffe pendant 2 heures à 140°C sous $10^{-2}$ mbar, 7,44 g de $CeCl_3,7H_2O$ finement broyé, ramène à température ambiante sous argon puis introduit 40 ml de THF/siliporite et agite la suspension 2 heures à température ambiante.

**2) Préparation du lithien**

On dissout à température ambiante sous azote 3,66 g de 2-(4-bromophényl)-1,3-dioxolane, dans 36 ml de THF/siliporite, puis ajoute en 20 minutes à -73°C ± 1°C, 10 ml de n-butyllithium, 1,6M et agite cette suspension pendant 3 h 30 à -75°C.

**3) Organo-cérien**

On mélange la suspension de $CeCl_3$ à la suspension de lithien et agite la suspension obtenue pendant 35 mn à -70°C.

**4) Addition nucléophile puis traitement acide**

Une solution de 1,408 g de 3,4-dihydro-6-méthoxy-1(2H)-naphtalénone dans 20 ml de THF est ajoutée à la suspension précédente, on agite 1 heure à -70°C, on ajoute 8 ml d'HCl et agite 1 h 30.

Après filtration, lavage, extraction à l'acétate d'éthyle, on évapore sous pression réduite jusqu'à obtention de 3,15 g de produit brut attendu que l'on purifie par chromatographie en éluant avec le mélange EP/AcOEt (9/1). On obtient 1,78 g de produit pur attendu.
Rf EP/AcOEt (9/1) = 0,22
F = 100°C
IR ($CHCl_3$)
C=O 1700 $cm^{-1}$ ; C=C + aromatique 1604, 1566, 1499 $cm^{-1}$.

Stade B : Bromation : 4-(2-bromo-3,4-dihydro-6-méthoxy-1-naphtalényl)-benzaldéhyde

A une solution de 1,78 g du produit obtenu au stade précédent, dans 18 ml de THF, sous azote et à température ambiante, on ajoute 2,15 g de perbromure de pyridinium à 95 %, agite cette suspension pendant 2 heures 30 à température ambiante, ajoute du bicarbonate de sodium, extrait à l'acétate d'éthyle, lave, sèche et évapore sous pression réduite jusqu'à obtention de 3,18 g de produit attendu.
Rf EP/AcOEt (9/1) = 0,22
IR ($CHCl_3$)
C=O 1703 $cm^{-1}$, C=C + aromatique 1604 (F), 1569, 1468 $cm^{-1}$.

Stade C : Arylation : 4-(3,4-dihydro-6-méthoxy-2-phényl-1-naphtalényl)-benzaldéhyde

On porte au reflux pendant 1 heure, le mélange constitué de 3,18 g dérivé bromé obtenu au stade précédent contenant 3/4 de mole de HO-$(CH_2)_3$-Br,

- 1,07 g d'acide phénylboronique, 230 mg de palladium tétrakis triphényl phosphine, 40 ml de THF, 20 ml d'eau et 1,82 g de bicarbonate de sodium, décante, lave, extrait à l'acétate d'éthyle, sèche et évapore sous pression réduite jusqu'à obtention de 3,2 g de produit brut que l'on purifie par chromatographie en éluant avec le mélange EP/AcOEt (9/1) pour obtenir 1,92 g de produit brut attendu.

Rf EP/AcOEt (9/1) = 0,23
F = 153°C
IR ($CHCl_3$)
C=O 1699 $cm^{-1}$ ; C=C + aromatique 1603, 1567, 1496 $cm^{-1}$.

Stade D : Réaction de wittig

A une solution de 1,02 g d'aldéhyde préparé au stade précédent dans 10 ml de THF, on ajoute à température ambiante et sous azote 1,15 g de carboéthoxyméthylène triphénylphosphorane et agite cette solution pendant 5 heures à température ambiante puis 17 heures au reflux.
Après évaporation sous pression réduite on obtient 2,2 g de produit brut que l'on purifie par chromatographie en éluant avec le mélange EP/AcOEt (8/2). On obtient 1,21 g de produit pur attendu.
Rf EP/AcOEt (8/2) = 0,07
F = 154°C.
IR ($CHCl_3$)
C=O 1705 $cm^{-1}$ ; C=C 1636 $cm^{-1}$ ; C=C + aromatique 1605 (max), 1567 (max), 1494 (max) ; absorption région H-C=C-H trans 1984 $cm^{-1}$.
RMN $CDCl_3$ 300 MHz $\delta$ en ppm
2,80 (m) et 2,96 (m) : $H_3$ et $H_4$ ; 1,33 (t) et 4,26 (q) : $CO_2$-C<u>H</u>$_2$-C<u>H</u>$_3$ ; 3,81 (s) : $OCH_3$ ; 7,64 (d,J=16) et 6,39 (d,J=16) : Ph-C<u>H</u>=C<u>H</u>-$CO_2$Et ; 6,68 (d,J=8,5) $H_1$ ; 6,59 (dd,J=2,5 et 8,5) $H_7$ ; 6,79 (d,J=2,5) $H_3$ ; 6,98 à 7,11 (m) : phényle en 2 ; 7,09 et 7,38 : phényle en 1.

**EXEMPLE 2 : 3-[4-(3,4-dihydro-6-hydroxy-2-phényl-1-naphtalényl)phényl]-2-propénoate d'éthyle**

A une solution de 776 mg du composé de l'exemple 1 dans 15 ml de dichlorométhane, on ajoute 4 fois 1,5 ml de $BF_3$,$Me_2S$ en maintenant à température ambiante, verse sur de l'eau salée, extrait, lave, sèche et évapore sous pression réduite jusqu'à obtention de 800 mg de produit attendu.
Rf EP/AcOEt (8/2) = 0,15

**EXEMPLE 3 : Acide 3-[4-(3,4-dihydro-6-hydroxy-2-phényl-1-naphtalényl)phényl]-propénoïque**

A une solution de 800 mg d'ester obtenu à l'exemple 2 dans 8 ml de méthanol, on ajoute 4 ml de soude 2N, chauffe pendant 15 mn à 50°C, ramène à température ambiante, acidifie avec HCl 2N, extrait à l'acétate d'éthyle, lave, sèche et évapore sous pression réduite jusqu'à obtention de 799 mg de produit brut que l'on purifie par chromatographie en éluant avec le mélange $CH_2Cl_2$/ MeOH (9/1) puis par recristallisation dans de l'éther isopropylique. On obtient 320 mg de produit pur attendu.
Rf $CH_2Cl_2$/MeOH (90/10) = 0,28

**EXEMPLE 4 : (+,-) cis 3-[4-(6-méthoxy-2-phényl-1,2,3,4-tétrahydro-1-naphtalényl)phényl]-2-propénoate d'éthyle**

Stade A : hydrogénation : (+,-) cis 4-(6-méthoxy-2-phényl-1,2,3,4-tétrahydro-1-naphtalényl)-phénol

A une solution de 268 mg de 4-(3,4-dihydro-6-méthoxy-2-phényl-1-naphtalényl)-phénol (préparé selon US 3947520) dans 2 ml d'éthanol et 2 ml d'acide acétique, on ajoute 30 mg de palladium sur charbon à 9,5 %, puis hydrogène à une température de 55°C, pendant 24 heures (3,5 bars). Après traitement, on obtient 235 mg de produit attendu.
Rf MeOH/$H_2O$ (9/1) = 0,25 (Whatman KC18)

F = 187°C.

Stade B : Activation du phénol : (+,-) cis trifluorométhane sulfonate de 4-(6-méthoxy-2-phényl-1,2,3,4-tétrahydro-1-naphtalényl)-phényle

A une solution de 1,77 g du produit obtenu au stade précédent dans 30 ml de pyridine, on ajoute goutte à goutte au bain de glace, 1,35 ml d'anhydride triflique et agite pendant 50 mn en laissant revenir à température ambiante. Après dilution avec de l'acétate d'éthyle, lavage à l'acide chlorhydrique 2N, au bicarbonate de sodium puis à l'eau, et séchage, on évapore sous pression réduite pour obtenir 2,53 g de produit brut que l'on purifie par chromatographie en éluant avec le mélange EP/AcOEt (95/5).
Rf EP/AcOEt (95/5) = 0,26
F = 130°C
IR ($CHCl_3$)
Absence d'OH
Aromatique 1610-1499 $cm^{-1}$.

Stade C : Couplage avec le stannane

On chauffe sous azote pendant 15 mn à 100°C le mélange constitué de

- 2,15 g de (E) 3-(tributylstannyl)-2-propénoate d'éthyle (préparé selon J.A.C.S. (1987) 109 815)
- 1262 mg de triflate préparé au stade précédent
- 12 ml de DMF
- 164 mg de $Pd(P\phi_3)_4$
- 349 mg de chlorure de lithium,

verse sur de l'eau, extrait à l'acétate d'éthyle, lave, sèche et évapore sous pression réduite jusqu'à obtention de 3,7 g de produit brut. Après redissolution dans l'acétate d'éthyle, on ajoute du fluorure de potassium, sèche, évapore sous pression réduite et purifie par chromatographie en éluant avec le mélange EP/AcOEt (9/1) puis avec le mélange $CH_3CN/H_2O$ (9/1) (Lichrosort RP18).
On obtient 613 mg de produit pur attendu.
Rf $CH_3CNH_2O$ (9/1) = 0,3
RMN : $CDCl_3$, 300 MHz, δ en ppm
1,31 (t) et 4,23 (q) : $CO_2$-C$\underline{H}_2$-C$\underline{H}_3$ ; 1,86 (dm) et 2,18 (m) : $H_3$ ; 3,00 à 3,20 (m) : $H_4$ ; 3,43 (ddd,J=3,5 et 13) : $H_2$ ; 4,31 (d,J=5) : $H_1$ ; 3,82 (s) : $OCH_3$ ; 7,56 (d,J=16) et 6,30 (d,J=16) : Ph-C$\underline{H}$=C$\underline{H}$-$CO_2$Et ; 6,44 et 7,15 : phényle en 1 ; 6,68 (dd) : $H_7$ ; 6,78 (d) : $H_5$ ; 6,84 (d) : $H_8$ ; 6,80 (m) et 7,15 (m) : : phényle en 2.

**EXEMPLE 5 : (+,-) cis 3-[4-(6-hydroxy-2-phényl-1,2,3,4-tétrahydro-1-naphtalényl)phényl]-2-propénoate d'éthyle**

On opère comme à l'exemple 2 mais à partir de 300 mg du produit de l'exemple 4. On obtient 300 mg de produit déméthoxylé attendu.
Rf EP/AcOEt (80/20) = 0,15

**EXEMPLE 6 : Acide (+,-) cis 3-[4-(6-hydroxy-2-phényl-1,2,3,4-tétrahydro-1-naphtalényl)phényl]-propénoique**

On opère comme à l'exemple 3, mais à partir de 300 mg de produit de l'exemple 5.
On obtient 235 mg de produit saponifié attendu.
Rf $CH_2Cl_2$/MeOH (9/1) = 0,3.
RMN $CDCl_3$ + $C_5D_5N$ - 300 MHz, δ en ppm
1,75 et 2,11 : $H_3$ ; 2,99 (m) : $H_4$ ; 3,38 (dm) : $H_2$ ; 4,30 (d,J=5) : $H_1$ ; 7,40 (d,J=16) et 6,36 (d,J=16) : Ph-C$\underline{H}$=C$\underline{H}$ $CO_2$Et ; 6,41 et 7,26 : phényle en 1 ; 6,50 (dd) : $H_7$ ; 6,65 : $H_5$ et $H_8$ ; 6,86 et 7,13 : phényle en 2.

**EXEMPLE 7 : (+,-) cis N,N-diéthyl-3-[4-(6-méthoxy-2-phényl-1,2,3,4-tétrahydro-1-naphtalényl)phényl]-2-propénamide**

Stade A : acide (+,-) cis 3-[4-(6-méthoxy-2-phényl-1,2,3,4-tétrahydro-1-naphtalényl)phényl] -2-propénoïque

On opère comme à l'exemple 3, mais à partir de 304 mg d'ester de l'exemple 4. On obtient 300 mg du produit

attendu. Rf $CH_2Cl_2$/MeOH (90/10) = 0,4.
RMN (DMSO), 300 MHz, δ en ppm
1,78 (m) et 2,13 (m) : $H_3$ ; 2,90 à 3,15 (m) : $H_4$ ; 3,40 (m) $H_2$ ; 4,35 (d,J=5) : $H_1$ ; 7,42 (d,J=16) et 6,36 (d,J=16) : Ph-C<u>H</u>=C<u>H</u>-COO ; 6,42 et 7,29 : phényle en 1 ; 6,66 (dd) : $H_7$ 6,77 (d) : $H_8$ ; 6,82 (d) : $H_5$ ; 6,87 (m) et 7,15 (m) : phényle en 2.

<u>Stade B</u> : (+,-) cis N,N-diéthyl-3-[4-(6-méthoxy-2-phényl-1,2,3,4-tétrahydro-1-naphtalényl)phényl]-2-propénamide

A une solution de 296 mg de l'acide obtenu au stade précédent dans 5 ml de dichlorométhane, on ajoute, à température ambiante et sous azote, 180 µl de méthylmorpholine, puis, à environ -10°C, 150 µl de chloroformiate d'isobutyle puis 320 µl de diéthylamine. Après agitation 30 mn à -10°C, on rajoute de l'acétate d'éthyle, lave avec de l'acide chlorhydrique 2N, au bicarbonate de sodium et à l'eau salée, sèche et évapore sous pression réduite jusqu'à obtention de 380 mg de produit attendu.
Rf EP/acétone (50/50) = 0,6.

<u>EXEMPLE 8</u> : (+,-) cis N,N-diéthyl-3-[4-(6-hydroxy-2-phényl-1,2,3,4-tétrahydro-1-naphtalényl)phényl]-2-propénamide

On opère comme à l'exemple 2 mais à partir de 380 mg d'amide préparée selon l'exemple 7. On obtient 240 mg de produit pur attendu.
Rf EP/acétone (6/4) = 0,35
F = 222°C.
IR (Nujol)
Absorption région OH/NH
C=O 1637 $cm^{-1}$ ; système conjugué + aromatique 1574, 1505, 1499 $cm^{-1}$.
RMN (DMSO) 300 MHz
1,04 (t) et 1,10 (t) ; ; 3,30 à 3,50 (m) : $CONEt_2$ ; 1,75 (dm) et 2,11 (m) $H_4$ ; 2,88 à 3,10 (m) $H_3$ ; 3,40 masqué $H_2$ ; 4,29 (d,J=5,5) $H_1$ ; 7,33 (d,J=15,5), 6,92 (d,J= 15,5) : Ph-C<u>H</u>=C<u>H</u>-CO ; 6,40 à 7,30 phényl en 1 ; 6,50 (dd) 1H, 6,65 (m) 2H, 6,87 (m) 2H, 7,14 (m) 3H aromatiques.

**<u>EXEMPLE 9</u> : 3-[4-[3,4-dihydro-6-méthoxy-2-(4-méthoxyphényl)-1-naphtalényl] phényl] 2-propénoate d'éthyle.**

<u>Stade A</u> : Arylation : 4-[3,4-dihydro-6-méthoxy-2-(4-méthoxyphényl) 1-naphtalényl] phénol.

On chauffe 1 heure au reflux le mélange constitué de 10,2 g de 4-(2-bromo 3,4-dihydro 6-méthoxy 1-naphtalényl) phénol, 4,9 g d'acide 4-méthoxy benzène boronique, 820 mg de palladium tétrakistriphénylphosphine, 100 ml de tétrahydrofuranne, 50 ml d'eau et 7,3 g de carbonate de sodium, décante, lave, extrait à l'acétate d'éthyle, sèche et évapore sous pression réduite jusqu'à obtention de 12,65 g de produit brut que l'on purifie par chromatographie en éluant avec le mélange EP-AcOEt 7-3) pour obtenir 8 g de produit brut attendu.
Rf : EP-AcOEt 9-1 = 0,23
F = 180°C.
IR ($CHCl_3$) : OH 3598 $cm^{-1}$ ; OMe 2837 $cm^{-1}$ ; C=C + aromatique 1608, 1568, 1494 cm-1.

<u>Stade B</u> : Activation du phénol : trifluorométhane sulfonate de 4-(6-méthoxy 2-(4-méthoxyphényl) 3,4-dihydro 1-naphtalényl) phényle.

On opère comme au stade B de l'exemple 4 en utilisant 1,79 g de produit obtenu au stade A, 18 ml de pyridine et 1,25 ml d'anhydride triflique. On obtient 2,26 g de produit attendu. F = 130°C.
IR ($CHCl_3$)
Absence d'OH ; C=C + aromatique 1608, 1566, 1511, 1498 $cm^{-1}$ ; $OSO_2CF_3$ 1423, 1141 $cm^{-1}$.

<u>Stade C</u> : Couplage avec le stannane : 3-[4-[3,4-dihydro-6-méthoxy-2-(4-méthoxyphényl)-naphtalényl] phényl] 2-propénoate d'éthyle.

On opère comme au stade C de l'exemple 4 en utilisant 2,25 g de (E) 3-(tributylstannyl) 2-propénoate d'éthyle [préparé selon J.A.C.S. (1987) <u>109</u>, 815], 2,69 g de triflate préparé au stade précédent, 25 ml de diméthylformamide, 275 mg de $Pd(P\phi_3)_4$ et 588 mg de chlorure de lithium. On obtient 1,4 g de produit attendu. F = 145°C.
Rf : 0,23 (EP-AcOEt 85-15)
IR ($CHCl_3$)

C=O 1705 $cm^{-1}$ ; C=C 1636 $cm^{-1}$ ; aromatique 1608, 1568, 1511, 1496 $cm^{-1}$.

**EXEMPLE 10 : 3-[4-[3,4-dihydro-6-hydroxy-2-(4-hydroxyphényl)-1-naphtalényl] phényl] 2-propénoate d'éthyle.**

On opère comme à l'exemple 2 en utilisant 948 mg de produit obtenu à l'exemple 9 et 4,2 ml de $BF_3$,$Me_2S$. On obtient 785 mg de produit brut que l'on chromatographie sur silice en éluant avec le mélange EP-AcOEt 1-1 et récupère 600 mg de produit pur. F = 214°C.
Rf = 0,37 (EP-AcOEt 1-1).

**EXEMPLE 11 : Acide 3-[4-[3,4-dihydro-6-hydroxy-2-(4-hydroxyphényl) 1-naphtalényl] phényl] 2-propénoïque.**

On opère comme à l'exemple 3 en utilisant au départ 365 mg de l'ester préparé à l'exemple 10. On obtient 326 mg de produit pur. F ≃ 180°C.
Rf = 0,3 ($CH_2Cl_2$-MeOH 85-15).

**EXEMPLE 12 : Acide 3-[4-[6-méthoxy-2-(4-méthoxyphényl)-3,4-dihydro 1-naphtalényl] phényl] 2-propénoique.**

On opère comme à l'exemple 7A en utilisant 440 mg de l'ester préparé à l'exemple 9. On obtient 406 mg de produit attendu. F = 206°C.

**EXEMPLE 13 : N,N-diéthyl 3-[4-[3,4-dihydro-6-méthoxy-2-(4-méthoxyphényl) 1-naphtalényl] phényl] 2-propénamide.**

On opère comme à l'exemple 7B en utilisant 400 mg de l'acide préparé à l'exemple 12, 0,23 ml de méthylmorpholine, 0,2 ml de chloroformiate d'isobutyle et 0,42 ml de diéthylamine. On obtient 557 mg de produit attendu utilisé tel quel pour l'exemple suivant.

**EXEMPLE 14 : N,N-diéthyl 3-[4-[3,4-dihydro-6-hydroxy-2-(4-hydroxyphényl) 1-naphtalényl] phényl] 2-propénamide.**

On opère comme à l'exemple 8 en utilisant 557 mg du composé préparé à l'exemple 13 et 2,1 ml de $BF_3$, $Me_2S$ et obtient 335 mg de produit attendu. F = 222°C.
Rf = 0,32 (EP-acétone 1-1).
RMN ($CDCl_3$) 200 MHz
1,19 (t)-1,26 (t)-3,49 (m) : $CONEt_2$ ; 2,75 (m) 2H et 2,92 (m) 2H : $H_3$ et $H_4$; 4,98 (s large) : : les OH ; 6,60 et 6,88 : phényl en 2 ; 6,79 (d,J=15,5)-7,67 (d,J=15,5) : Ph-C<u>H</u>=C<u>H</u>-CO ; 7,06 et 7,37 : phényl en 1.

**EXEMPLE 15 : (+,-) cis 3-[4-[6-méthoxy-2-(4-méthoxyphényl) 1,2,3,4-tétrahydro 1-naphtalényl] phényl] 2-propénoate d'éthyle.**

En opérant comme à l'exemple 4, stades A, B, C à partir du phénol préparé à l'exemple 9, stade A, on a préparé le produit attendu.
Rf : 0,24 (EP-AcOEt 85-15)
IR ($CHCl_3$)
OMe 2838 $cm^{-1}$ ; C=O 1704 $cm^{-1}$ ; C=C conj. 1636 $cm^{-1}$ ; aromatiques 1609 (F), 1513 (F), 1501 (F) $cm^{-1}$ ; C=C ΔE 984 $cm^{-1}$.

**EXEMPLE 16 : (+,-) cis 3-[4-[6-hydroxy-2-(4-hydroxyphényl) 1,2,3,4-tétrahydro 1-naphtalényl] phényl] 2-propénoate d'éthyle.**

On opère comme à l'exemple 2 en utilisant 313 mg de produit obtenu à l'exemple 15 et 1,4 ml de $BF_3$,$Me_2S$. On obtient 299 mg de produit brut que l'on chromatographie sur silice (éluant EP-AcOEt 6-4) et récupère 244 mg de produit pur.
Rf = 0,30 (EP-AcOEt 6-4).
IR (Nujol)
OH 3418 $cm^{-1}$ ; C=O 1685 $cm^{-1}$ ; aromatique 1620, 1612, 1587, 1514 (F), 1493 $cm^{-1}$ ; C=C ΔE def. 980 $cm^{-1}$.

**Exemple 17 : (+,-) cis acide 3-[4-[6-méthoxy-2-(4-méthoxyphényl) 1,2,3,4-tétrahydro 1-naphtalényl] phényl] 2-propénoïque.**

On opère comme à l'exemple 3, mais à partir de 1,3 g d'ester de l'exemple 15. On obtient 1,2 g du produit attendu. Rf = 0,4 ($CH_2Cl_2$-MeOH 90-10).
RMN (DMSO), 300 MHz, Δ en ppm
1,82 (dl) et 2,12 (m) : $H_3$ ; = 3,10 (ml) : $H_4$ ; 3,38 (ddd) : $H_2$ ; 4,28 (d, J=5) : $H_1$ ; 7,66 (d, J=16) et 6,30 (d, J=16) : Ph-CH=CH-COO ; 6,47 et 7,18 : phényle en 1 ; 6,68 : $H_7$ ; 6,89 (d, J=9) : $H_8$ ; 6,78 (d, J=2) : $H_5$ ; 6,72 : phényle en 2.

**EXEMPLE 18 : (+,-) cis 3-[4-[6-hydroxy-2-(4-hydroxyphényl) 1,2,3,4-tétrahydro 1-naphtalényl] phényl] 2-propénoïque.**

On chauffe à 160°C pendant 4 heures et demie 270 mg d'acide préparé à l'exemple 17 en présence de 3 g de chlorhydrate de pyridine. On laisse revenir à température ambiante, extrait à l'acétate d'éthyle, lave avec de l'acide chlorhydrique 2N, puis à l'eau et concentre. On obtient 309 mg de produit brut que l'on chromatographie sur silice (éluant : $CH_2Cl_2$-MeOH 85-15) et obtient 177 mg de produit attendu.
Rf = 0,3 ($CH_2Cl_2$-MeOH 85-15).
IR (Nujol)
absorption générale : OH/NH ; C=O : 1622 $cm^{-1}$ ; C=C + aromatique : 1629, 1605, 1560, 1513, 1499 $cm^{-1}$.

**EXEMPLE 19 : (+,-) cis N,N-diéthyl 3-[4-[6-méthoxy-2-(4-méthoxyphényl) 1,2,3,4-tétrahydro 1-naphtalényl] phényl] 2-propénamide.**

On opère comme à l'exemple 7B en utilisant 300 mg de l'acide préparé à l'exemple 17, 0,16 ml de méthylmorpholine, 0,14 ml de chloroformiate d'isobutyle et 0,22 ml de diéthylamine. On obtient 425 mg de produit attendu utilisé tel quel pour l'exemple suivant.

**EXEMPLE 20 : (+,-) cis N,N-diéthyl 3-[4-[6-hydroxy-2-(4-hydroxyphényl) 1,2,3,4-tétrahydro 1-naphtalényl] phényl] 2-propénamide.**

On opère comme à l'exemple 8 en utilisant 425 mg du composé préparé à l'exemple 19 et 1,4 ml de $BF_3$, $Me_2S$ et obtient 202 mg de produit attendu.
Rf = 0,30 (EP-acétone 1-1).
RMN (DMSO), 300 MHz Δ en ppm
1,05 (t)-1,11 (t)- = 3,32 (m)-3,46 (m) : $CONEt_2$ ; 1,69 (m) et 2,02 (m) : $H_3$ ; 2,97 (m) : $H_4$ ; 3,26 (m) : $H_2$ ; 4,21 (d, J=5): $H_1$ ; 7,34 (d, J=15,5) et 6,93 (d, J=15,5) : Ph-CH=CH- ; 6,41 et 7,31, 6,54 et 6,63 : les phényles en 1 et 2 ; 6,48 (dd) : $H_7$ ; 6,60 à 6,69 : $H_5$.

En opérant comme dans les exemples précédents, on a préparé les produits des exemples suivants :

**EXEMPLE 21 : 3-[4-[3,4-dihydro 6-méthoxy-2-(4-fluorophényl) 1-naphtalényl] phényl] 2-propénoate d'éthyle.**

Stade A : Arylation : 4-[3,4-dihydro 6-méthoxy-2-(4-fluorophényl) 1-naphtalényl] phénol.

F = 142°C.
Rf = 0,26 (EP-AcOEt 8-2).
IR ($CHCl_3$)
OH : 3596 $cm^{-1}$ ; C=C + aromatique : 1605, 1591, 1568, 1507, 1495 $cm^{-1}$.

Stade B : Activation du phénol : trifluorométhane sulfonate de 4-[6-méthoxy-2-(4-fluorophényl) 3,4-dihydro 1-naphtalényl] phényle.

Rf = 0,35 (Ep-AcOEt 9-1)
IR ($CHCl_3$)
C=C + aromatique : 1606, 1568, 1508, 1499 $cm^{-1}$ ; $OSO_2CF_3$ : 1424 et 1141 $cm^{-1}$.

Stade C : Couplage avec le Stannane : 3-[4-[3,4-dihydro 6-méthoxy-2-(4-fluorophényl) 1-naphtalényl] phényl] 2-propénoate d'éthyle.

Rf = 0,3 (cyclohexanne-AcOEt 85-15)
IR ($CHCl_3$)
C=O : 1705 $cm^{-1}$ ester conjugué ; C=C : 1637 $cm^{-1}$ ; aromatique: 1606, 1569, 1508, 1497 $cm^{-1}$.

**EXEMPLE 22 : 3-[4-[3,4-dihydro 6-hydroxy-2-(4-fluorophényl) 1-naphtalényl] phényl] 2-propénoate d'éthyle.**

F = 196°C.
Rf = 0,27 (EP-AcOEt 7-3)
IR ($CHCl_3$)
OH : 3595 $cm^{-1}$ ; C=O : 1706 $cm^{-1}$ ; C=C : 1637 $cm^{-1}$ ; aromatique : 1605, 1590, 1575, 1558, 1508, 1497 $cm^{-1}$.

**EXEMPLE 23 : acide 3-[4-[3,4-dihydro 6-hydroxy-2-(4-fluorophényl) 1-naphtalényl] phényl]-propénoïque.**

Rf = 0,25 ($CH_2Cl_2$-MeOH 9-1)
IR (Nujol)
C=O : 1684 $cm^{-1}$ ; C=C : 1629 $cm^{-1}$ ; aromatique : 1603, 1574, 1505 $cm^{-1}$.

**EXEMPLE 24 : acide 3-[4-[6-méthoxy-2-(4-fluorophényl) 3,4-dihydro 1-naphtalényl] phényl] 2-propénoïque.**

Rf = 0,37 ($CHCl_2$-MeOH 9-1)
RMN (DMSO), 300 MHz ppm
3,75 (s) : MeO ; ≃ 2,70 ≃ 2,93 :$H_3$ et $H_4$ ; 6,49 (d, J=15,5) et 7,55 (d, J=15,5) : $\phi$-$CH_2$=$CH_2$-CO.

**EXEMPLE 25 : N,N-diéthyl 3-[4-[3,4-dihydro 6-méthoxy-2-(4-fluorophényl) 1-naphtalényl] phényl] 2-propénamide.**

**EXEMPLE 26 : N,N-diéthyl 3-[4-[3,4-dihydro 6-hydroxy-2-(4-fluorophényl) 1-naphtalényl] phényl] 2-propénamide.**

F ≃ 240°C.
Rf = 0,33 (EP-Acétone 6-4).
IR (Nujol)
absorption générale : OH/NH ; C=O : 1640 $cm^{-1}$ ; C=C + aromatique : 1605, 1574, 1502, 1484 $cm^{-1}$.

**EXEMPLE 27 : (+,-) cis 3-[4-[6-méthoxy-2-(4-fluorophényl) 1,2,3,4-tétrahydro 1-naphtalényl] phényl] 2-propénoate d'éthyle.**

Rf = 0,27 (cyclohexanne-AcOEt 9-1)
IR ($CHCl_3$)
C=O : 1705 $cm^{-1}$ ; C=C : 1637 $cm^{-1}$ ; aromatique : 1608, 1578, 1565, 1510, 1501 $cm^{-1}$.

**EXEMPLE 28 : (+,-) cis 3-[4-[6-hydroxy-2-(4-fluorophényl) 1,2,3,4-tétrahydro 1-naphtalényl] phényl] 2-propénoate d'éthyle.**

Rf = 0,28 (EP-AcOEt 7-3).
IR ($CHCl_3$)
OH : 3599 $cm^{-1}$ ; C=O : 1703 $cm^{-1}$ ; C=C : 1637 $cm^{-1}$ ; aromatique : 1607, 1585, 1565, 1510, 1499 $cm^{-1}$.

**EXEMPLE 29 : (+,-) cis acide 3-[4-[2-(4-fluorophényl) 6-hydroxy 1,2,3,4-tétrahydro 1-naphtalényl] phényl] 2-propénoïque.**

Rf = 0,28 ($CH_2Cl_2$-MeOH 9-1).
IR (Nujol)
absorption région : OH/NH ; C=O : 1682 $cm^{-1}$ ; C=C + aromatique : 1626, 1600, 1560, 1500 $cm^{-1}$.

**EXEMPLE 30 : (+,-) cis acide 3-[4-[2-(4-fluorophényl) 6 méthoxy 1,2,3,4-tétrahydro 1-naphtalényl] phényl] 2-propénoïque.**

Rf = 0,4 ($CH_2Cl_2$-MeOH 9-1).
IR ($CHCl_3$)
absorption OH type acide ; C=O : 1688 $cm^{-1}$ ; C=C : 1630 $cm^{-1}$ aromatique : 1607, 1576 (f), 1563 (f), 1510, 1501 $cm^{-1}$.

**EXEMPLE 31 : (+,-) cis N,N-diéthyl 3-[4-[6-méthoxy-2-(4-fluorophényl) 1,2,3,4-tétrahydro 1-naphtalényl] phényl] 2-propénamide.**

Rf = 0,48 (EP-Acétone 6-4).

**EXEMPLE 32 : (+,-) cis N,N-diéthyl 3-[4-[6-hydroxy-2-(4-fluorophényl) 1,2,3,4-tétrahydro 1-naphtalényl] phényl] 2-propénamide.**

F = 260°C.
Rf = 0,32 (EP-Acétone 6-4).
IR (Nujol)
absorption générale : OH/NH ; C=O : 1640 $cm^{-1}$ ; C=C + aromatique : 1600, 1580, 1508, 1498 $cm^{-1}$.

Tests pharmacologiques

**1 - Effet sur la prolifération de cellules mammaires**

L'activité proliférative des molécules est étudiée comparativement à celle de l'oestradiol sur les cellules mammaires humaines MCF-7 en culture.

Pour mettre en évidence un effet agoniste de l'oestradiol et/ou des molécules testées, le milieu de culture d'entretien des cellules (riche en facteurs de croissance et en stéroïdes) est remplacé par un milieu appauvri, entre autres dépourvu de stéroïdes (DMEM supplémenté par 5 % de sérum déstéroïdé et sans rouge de phénol). Les cellules subissent ce sevrage deux jours avant le début de l'essai.

Après 7 jours de culture en présence des produits à étudier, la prolifération cellulaire est évaluée par dosage du DNA. Dans chaque essai, l'effet de l'oestradiol à $10^{-10}$M (croissance cellulaire en présence d'oestradiol moins croissance cellulaire en présence du solvant) détermine le 100 % de l'activité agoniste. L'activité des molécules est évaluée en comparaison à ce témoin interne. Les molécules induisant une croissance cellulaire identique à celle observée avec le solvant seul sont classées "inactives", celles induisant une croissance cellulaire inférieure à celle observée avec le solvant sont classées "inhibiteur".

| | ACTIVITE |
|---|---|
| Estradiol | |
| Exemple 3 | Inhibiteur |
| Exemple 6 | Inhibiteur |
| Exemple 8 | Inhibiteur |

Conclusion :

Les produits testés ne sont pas agonistes de la croissance des cellules MCF-7, certains sont même inhibiteurs de celle-ci.

**2 - Affinité au récepteur estrogène humain (REH)**

Un extrait cytosolique de cellules SF9 contenant le récepteur oestrogène humain recombinant est obtenu par surexpression dans un système cellules d'insectes-Baculovirus, selon la méthodologie générale décrite par N.R. WEBB et al. (Journal of Methods in Cell and Molecular Biology, (1990) vol 2 n° 4, 173-188) et dont l'application est décrite pour l'expression des récepteurs hormonaux humains, par exemple le récepteur glucocorticoïde humain (G. SRINIVASAN et al. Molecular Endocrinology (1990) vol 4 n° 2 209-216).

On utilise le kit BaculoGold Transfection Kit (PharMingen, référence 21000K) pour générer le baculovirus recombinant contenant le fragment d'ADNc décrit dans le vecteur d'expression HEGO par L. TORA et al. (The EMBO Journal (1989) vol 8 n° 7 1981-1986), comprenant la région codante pour le récepteur estrogène humain de type sauvage avec une glycine en position 400.

Le virus recombinant ainsi obtenu est utilisé pour exprimer le récepteur progestogène dans les cellules d'insectes SF9 (ATCC CRL1711), selon la méthodologie connue citée précédemment.

2 x $10^7$ cellules SF9 sont cultivées dans un flacon "Falcon" de 175 $cm^2$ dans le milieu TNM-FH "SIGMA" supplémenté avec 10 % de sérum de veau foetal (SVF) et avec 50 microgrammes/ml de gentamycine. Après infection puis incubation à 27°C pendant 40 à 42 heures, les cellules sont lysées dans 1 ml de tampon de lyse (Tris 20 mM-HCl pH8, EDTA 0,5 mM, DTT 2 mM, Glycérol 20 %, KCl 400 mM) par un cycle de congélation-décongélation que l'on répète encore deux fois. Le surnageant, contenant le récepteur estrogène humain recombinant est conservé dans l'azote liquide par dose de 0,5 ml.

Le surnageant est incubé à 0°C pendant 24 heures avec une concentration constante (T) d'oestradiol tritié en présence de concentrations croissantes soit d'estradiol froid (0-1000 x $10^{-9}$M), soit du produit froid à tester (0 - 25000 x $10^{-9}$M). La concentration d'estradiol tritié liée (B) est ensuite mesurée dans chaque incubat par la technique d'adsorption au charbon dextran.

**3 - Calcul de l'affinité relative de liaison (ARL)**

On trace les 2 courbes suivantes : le pourcentage de l'hormone tritiée liée 100xB/BO en fonction du logarithme de la concentration de l'hormone de référence froide ou en fonction du logarithme de la concentration du produit froid testé.

On détermine la droite d'équation suivante :

$$I_{50} = 100\ (B_0/B_0+Bmin/B_0)/2 = 100\ (1+Bmin/B_0)=50(1+Bmin/B_0)$$

$B_0$ = Concentration de l'hormone tritiée liée en l'absence de tout produit froid,
B = Concentration de l'hormone tritiée liée en présence d'une concentration X de produit froid,
Bmin = Concentration de l'hormone tritiée liée pour une incubation de cette hormone tritiée à la concentration (T) en présence d'un grand excès d'hormone froide de référence (1000x$10^{-9}$M) pour récepteur humain.

Les intersections de la droite $I_{50}$ et des courbes, permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminé par l'équation :

$$ARL = 100\ (CH)/(CX)$$

Les résultats obtenus sont les suivants :

| EXEMPLES | EH estradiol = 100 24 H |
|---|---|
| 3 | 72 |
| 6 | 39 |
| 8 | 5 |

Conclusion :

Les produits testés présentent une bonne affinité pour le récepteur estrogène humain.

## Revendications

1. Composés de formule générale (I) :

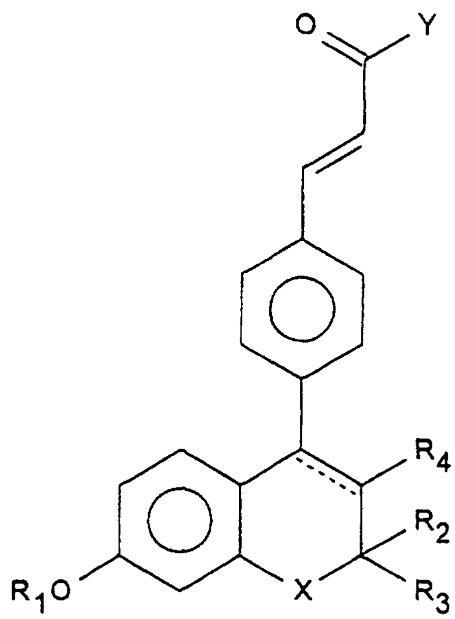

(I)

dans laquelle :

$R_1$ représente un atome d'hydrogène, un radical alkyle ou un radical acyle,
$R_2$ et $R_3$, identiques ou différents, représentent un atome d'hydrogène, un radical alkyle, ou forment ensemble un cycloalkyle renfermant de 3 à 7 atomes de carbone,
$R_4$ représente un radical aryle ou hétéroaryle,
X représente O ou $CH_2$,
Y représente un radical hydroxy, alkyloxy ou un groupement NRaRb dans lequel :

soit Ra et Rb, identiques ou différents, représentent un atome d'hydrogène ou un radical alkyle,
soit Ra et Rb forment ensemble avec l'atome d'azote auquel ils sont liés un hétérocycle mono ou polycyclique de 3 à 15 chaînons renfermant éventuellement un hétéroatome additionnel choisi parmi l'oxygène, le soufre et l'azote, lesdits alkyle, alkyloxy et acyle renferment de 1 à 6 atomes de carbone, lesdits alkyle, acyle, aryle et hétérocycle sont substitués ou non substitués,
le trait en pointillé représente éventuellement une double liaison,
les composés de formule (I) pouvant être sous toutes leurs formes stéréoisomères possibles, isolés ou en mélanges, ainsi que les sels d'additions avec les acides ou les bases.

**2.** Composés de formule (I) selon la revendication 1, dans laquelle : $R_1$, $R_2$ et $R_3$ sont des atomes d'hydrogène, $R_4$ est un phényle substitué ou non substitué et Y représente un radical hydroxy, alkyloxy renfermant de 1 à 6 atomes de carbone ou NRaRb, Ra et Rb représentant chacun un radical alkyle renfermant de 1 à 6 atomes de carbone.

**3.** Composés de formule (I) selon la revendication 1 ou 2, dans laquelle les groupements $R_4$ et Ph-CH=CH-COY sont en position cis.

**4.** Composés de formule (I) selon la revendication 1, suivants :

- acide 3-[4-(3,4-dihydro-6-hydroxy-2-phényl-1-naphtalényl) phényl]-2-propénoïque,
- acide (+,-) cis 3-(4-(6-hydroxy-2-phényl-1,2,3,4-tétrahydro-1-naphtalényl)phényl]-2-propénoïque,
- (+,-) cis N,N-diéthyl-3-[4-(6-hydroxy-2-phényl-1,2,3,4-tétrahydro-1-naphtalényl)phényl]-2-propénamide,
- 3-[4-(3,4-dihydro-6-méthoxy-2-(4-méthoxyphényl)-naphtalényl) phényl] 2-propénoate d'éthyle,
- 3-[4-(3,4-dihydro-6-hydroxy-2-(4-hydroxyphényl) 1-naphtalényl) phényl] 2-propénoate d'éthyle,
- acide 3-[4-(3,4-dihydro-6-hydroxy-2-(4-hydroxyphényl)-naphtalényl) phényl] 2-propénoïque,
- acide 3-[4-(6-méthoxy-2-(4-méthoxyphényl) 3,4-dihydro 1-naphtalényl) phényl] 2-propénoïque,
- N,N-diéthyl 3-[4-[3,4-dihydro-6-méthoxy-2-(4-méthoxyphényl) 1-naphtalényl] phényl] 2-propenamide,
- N,N-diéthyl 3-[4-[3,4-dihydro-6-hydroxy-2-(4-hydroxyphényl) 1-naphtalényl] phényl] 2-propénamide,

- (+,-) cis 3-[4-[6-méthoxy-2-(4-méthoxyphényl) 1,2,3,4-tétrahydro 1-naphtalényl) phényl] 2-propénoate d'éthyle,
- (+,-) cis 3- [4- [6-hydroxy-2- (4-hydroxyphényl) 1,2,3,4-tétrahydro 1-naphtalényl] phényl] 2-propénoate d'éthyle,
- acide (+,-) cis 3-[4-[3,4-[6-méthoxy-2-(4-méthoxyphényl)] 1,2,3,4-tétrahydro-1-naphtalényl] phényl] 2-propénoîque,
- acide (+,-) cis 3-[4-[6-hydroxy-2-(4-hydroxyphényl) 1,2,3,4-tétrahydro 1-naphtalényl] phényl] 2-propénoïque,
- (+,-) cis N,N-diéthyl-3-[4-[6-méthoxy-2-(4-méthoxyphényl) 1,2,3,4-tétrahydro 1-naphtalényl] phényl] 2-propénamide,
- (+,-) cis N,N-diéthyl-3-[4-[6-hydroxy-2-(4-hydroxyphényl) 1,2,3,4-tétrahydro 1-naphtalényl] phényl] 2-propénamide,
- 3-[4-[3,4-dihydro-6-méthoxy-2-(4-fluorophényl) 1-naphtalényl) phényl] 2-propénoate d'éthyle,
- 3-[4- [3,4-dihydro-6-hydroxy-2-(4-fluorophényl) 1-naphtalényl) phényl] 2-propénoate d'éthyle,
- acide 3-[4-[3,4-dihydro-6-hydroxy-2-(4-fluorophényl) 1-naphtalényl) phényl] 2-propénoïque,
- acide 3-[4-[6-méthoxy-2-(4-fluorophényl) 3,4-dihydro 1-naphtalényl] phényl] 2-propénoïque,
- N,N-diéthyl 3-[4-[3,4-dihydro-6-méthoxy-2-(4-fluorophényl) 1-naphtalényl] phényl] 2-propénamide,
- N,N-diéthyl 3-[4-[3,4-dihydro-6-hydroxy-2-(4-fluorophényl) 1-naphtalényl] phényl] 2-propénamide,
- (+,-) cis 3-[4-[6-méthoxy-2-(4-fluorophényl) 1,2,3,4-tétrahydro 1-naphtalényl] phényl] 2-propénoate d'éthyle,
- (+,-) cis 3-[4-[6-hydroxy-2-(4-fluorophényl) 1,2,3,4-tétrahydro 1-naphtalényl] phényl] 2-propénoate d'éthyle,
- (+,-) cis acide 3-[4-[2-(4-fluorophényl) 6-hydroxy 1,2,3,4-tétrahydro 1-naphtalényl] phényl] 2-propénoïque,
- (+,-) cis acide 3-[4-[2-(4-fluorophényl) 6-méthoxy 1,2,3,4-tétrahydro 1-naphtalényl] phényl] 2-propénoïque,
- (+,-) cis N,N-diéthyl 3-[4-[6-méthoxy-2-(4-fluorophényl) 1,2,3,4-tétrahydro 1-naphtalényl] phényl] 2-propénamide,
- (+,-) cis N,N-diéthyl 3-[4- [6-hydroxy-2- (4-fluorophényl) 1,2,3,4-tétrahydro 1-naphtalényl] phényl] 2-propénamide,
- 3-[4-(3,4-dihydro-6-méthoxy-2-phényl-1-naphtalényl) phényl] 2-propénoate d'éthyle,
- 3-[4-(3,4-dihydro-6-hydroxy-2-phényl-1-naphtalényl) phényl] 2-propénoate d'éthyle,
- (+,-) cis 3-[4-(6-méthoxy-2-phényl) 1,2,3,4-tétrahydro-1-naphtalényl) phényl] 2-propénoate d'éthyle,
- (+,-) cis 3-[4-(6-hydroxy-2-phényl-1,2,3,4-tétrahydro-1-naphtalényl) phényl] 2-propénoate d'éthyle,
- (+,-) cis N,N-diéthyl 3-[4-(6-méthoxy-2-phényl-1,2,3,4-tétrahydro 1-naphtalényl) phényl] 2-propénamide,

ces composés de formule (I) pouvant être sous toutes leurs formes stéréoisomères possibles isolées ou en mélange ainsi que les sels d'addition avec les acides et les bases.

**5.** Procédé de préparation des composés de formule (I) selon la revendication 1, **caractérisé en ce que** l'on soumet un composé de formule (II) :

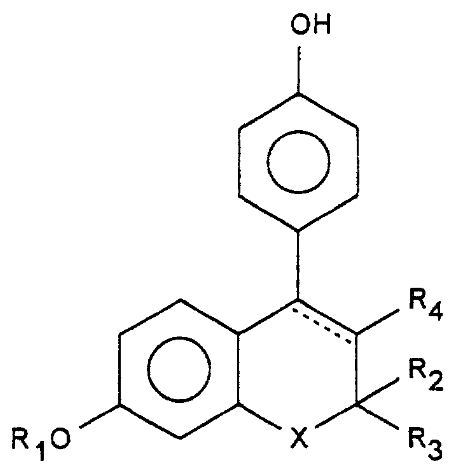

(II)

dans laquelle $R_1$, $R_2$, $R_3$, $R_4$ et X sont tels que définis à la revendication 1, à l'action d'un agent activant du phénol, puis à l'action d'un composé de formule :

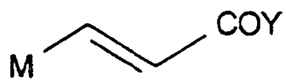

(III)

dans laquelle M est un dérivé organométallique, de préférence organostannique, et Y est tel que défini à la revendication 1, afin d'obtenir le composé de formule (I) qui le cas échéant, si désiré ou si nécessaire et dans un ordre approprié est mis en oeuvre dans l'une ou plusieurs des réactions suivantes :

- alkylation/déalkylation en position 6,
- acylation/clivage de l'acyloxy en position 6,
- estérification/saponification de l'acide ou de l'ester que représente COY,
- amidification de l'acide ou de l'ester que représente COY,
- salification
- séparation des diastéréoisomères, résolution et/ou transformation en un autre isomère.

**6.** Procédé de préparation des composés de formule (I) selon la revendication 1, dans laquelle le trait en pointillé représente une double liaison, **caractérisé en ce que** l'on soumet un composé de formule (IV) :

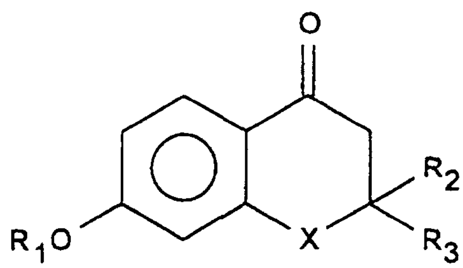

(IV)

$R_1$, $R_2$, $R_3$ et X étant tels que définis à la revendication 1, à l'action d'un organo-métallique magnésium ou lithium dérivé du 4-halogéno benzaldéhyde protégé, de préférence en présence de chlorure de cérium, afin d'obtenir, après traitement acide, le composé de formule (V) :

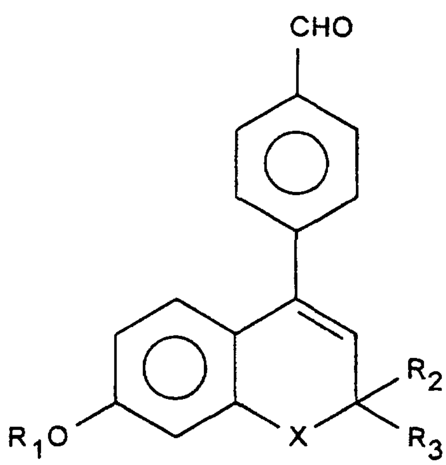

(V)

composé de formule (V) que l'on soumet à l'action d'un réactif d'halogénation afin d'obtenir un composé de formule (VI) :

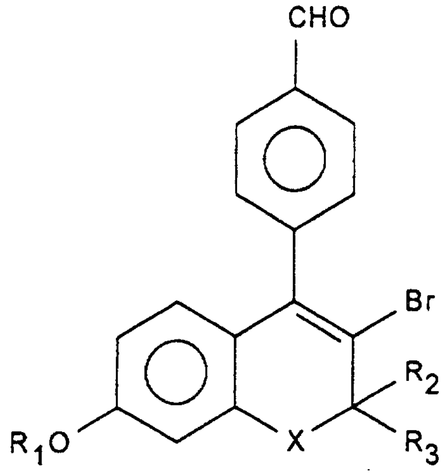

(VI)

composé de formule (VI) que l'on soumet à l'action d'un réactif d'arylation, afin d'obtenir un composé de formule (VII) :

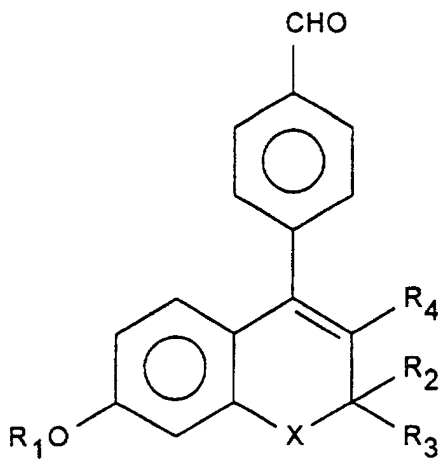

(VII)

composé de formule (VII) que l'on soumet à l'action d'un réactif de wittig de formule :

$$\phi_3P{=}CH\text{-}COY$$

dans laquelle Y est tel que défini à la revendication 1, afin d'obtenir le composé de formule (I) qui, le cas échéant, si désiré et si nécessaire et dans un ordre approprié est mis en oeuvre dans l'une ou plusieurs des réactions suivantes :

- alkylation/déalkylation en position 6,
- acylation/clivage de l'acyloxy en position 6,
- estérification/saponification de l'acide ou de l'ester que représente COY,
- amidification de l'acide ou de l'ester que représente COY,
- salification
- séparation des diastéréoisomères, résolution et/ou transformation en un autre isomère.

**7.** Composés de formule (I) telle que définie à l'une quelconque des revendications 1 à 3, à titre de médicaments.

**8.** Composés tels que définis à la revendication 4, à titre de médicaments.

**9.** Compositions pharmaceutiques renfermant au moins un médicament tel que défini à la revendication 7 ou 8, ainsi qu'un ou plusieurs excipients pharmaceutiquement acceptables.

**10.** Composés de formules (V), (VI) ou (VII) telles que définies à la revendication 6.

**Patentansprüche**

**1.** Verbindungen der allgemeinen Formel (I)

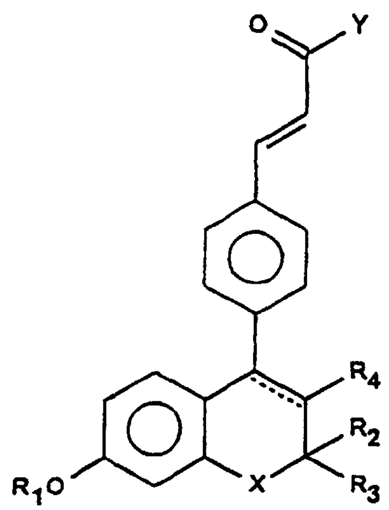

(I)

in der

$R_1$ ein Wasserstoffatom, einen Rest Alkyl oder einen Rest Acyl darstellt,
$R_2$ und $R_3$, gleich oder verschieden, ein Wasserstoffatom oder einen Rest Alkyl bedeuten oder zusammen ein Cycloalkyl mit 3 bis 7 Kohlenstoffatomen bilden,
$R_4$ einen Rest Aryl oder Heteroaryl darstellt,
X O oder $CH_2$ ist,
Y einen Rest Hydroxy, Alkyloxy oder eine Gruppe NRaRb bedeutet, worin:

entweder Ra und Rb, gleich oder verschieden, ein Wasserstoffatom oder einen Rest Alkyl darstellen, oder Ra und Rb zusammen mit dem Stickstoffatom, an das sie gebunden sind, einen mono- oder polycyclischen Heterocyclus mit 3 bis 15 Ringgliedern bilden, der gegebenenfalls ein zusätzliches Heteroatom umfaßt, ausgewählt unter Sauerstoff, Schwefel und Stickstoff, wobei die genannten Alkyl, Alkyloxy und Acyl 1 bis 6 Kohlenstoffatome umfassen, und
die genannten Alkyl, Acyl, Aryl und Heterocyclen substituiert oder nicht substituiert sind,
die punktierte Linie gegebenenfalls eine Doppelbindung darstellt, wobei die Verbindungen der Formel (I) in allen ihren möglichen stereoisomeren Formen, isoliert oder in Mischung, vorliegen können, sowie die Additionssalze mit Säuren oder Basen.

**2.** Verbindungen der Formel (I) nach Anspruch 1, worin $R_1$, $R_2$ und $R_3$ Wasserstoffatome sind, $R_4$ ein Phenyl ist, substituiert oder nicht substituiert, und Y einen Rest Hydroxy, Alkyloxy mit 1 bis 6 Kohlenstoffatomen oder NRaRb darstellt, worin Ra und Rb jeweils einen Rest Alkyl mit 1 bis 6 Kohlenstoffatomen bedeuten.

**3.** Verbindungen der Formel (I) nach Anspruch 1 oder 2, worin sich die Gruppen $R_4$ und Ph-CH=CH-COY in Position cis befinden.

**4.** Verbindungen der Formel (I) nach Anspruch 1, die folgen:

- 3-[4-(3,4-Dihydro-6-hydroxy-2-phenyl-1-naphthalenyl)-phenyl]-2-propensäure,
- (+,-) cis-3-[4-(6-Hydroxy-2-phenyl-1,2,3,4-tetrahydro-1-naphthalenyl)-phenyl]-2-propensäure,

- (+,-) cis-N,N-Diethyl-3-[4-(6-hydroxy-2-phenyl-1,2,3,4-tetrahydro-1-naphthalenyl)-phenyl]-2-propenamid,
- 3-{4-[3,4-Dihydro-6-methoxy-2-(4-methoxyphenyl)-naphthalenyl]-phenyl}-2-propensäure-ethylester,
- 3-{4-[3,4-Dihydro-6-hydroxy-2-(4-hydroxyphenyl)-1-naphthalenyl]-phenyl}-2-propensäure-ethylester,
- 3-{4-[3,4-Dihydro-6-hydroxy-2-(4-hydroxyphenyl)-naphthalenyl]-phenyl}-2-propensäure,
- 3-{4-[3,4-Dihydro-6-methoxy-2-(4-methoxyphenyl)-1-naphthalenyl]-phenyl}-2-propensäure,
- N,N-Diethyl-3-{4-[3,4-dihydro-6-methoxy-2-(4-methoxyphenyl)-1-naphthalenyl]-phenyl}-2-propenamid,
- N,N-Diethyl-3-{4-[3,4-dihydro-6-hydroxy-2-(4-hydroxyphenyl)-1-naphthalenyl]-phenyl}-2-propenamid,
- (+,-) cis-3-{4-[6-Methoxy-2-(4-methoxyphenyl)-1,2,3,4-tetrahydro-1-naphthalenyl]-phenyl}-2-propensäure-ethylester,
- (+,-) cis-3-{4-[6-Hydroxy-2-(4-hydroxyphenyl)-1,2,3,4-tetrahydro-1-naphthalenyl]-phenyl}-2-propensäure-ethylester,
- (+,-) cis-3-{4-[3,4-[6-Methoxy-2-(4-methoxyphenyl)]-1,2,3,4-tetrahydro-1-naphthalenyl]-phenyl}-2-propensäure,
- (+,-) cis-3-{4-[6-Hydroxy-2-(4-hydroxyphenyl)-1,2,3,4-tetrahydro-1-naphthalenyl]-phenyl}-2-propensäure,
- (+,-) cis-N,N-Diethyl-3-{4-[6-methoxy-2-(4-methoxyphenyl)-1,2,3,4-tetrahydro-1-naphthalenyl]-phenyl}-2-propenamid,
- (+,-) cis-N,N-Diethyl-3-{4-[6-hydroxy-2-(4-hydroxyphenyl)-1,2,3,4-tetrahydro-l-naphthalenyl]-phenyl}-2-propenamid,
- 3-{4-[3,4-Dihydro-6-methoxy-2-(4-fluorphenyl)-1-naphthalenyl]-phenyl}-2-propensäure-ethylester,
- 3-{4-[3,4-Dihydro-6-hydroxy-2-(4-fluorphenyl)-1-naphthalenyl]-phenyl}-2-propensäure-ethylester,
- 3-{4-[3,4-Dihydro-6-hydroxy-2-(4-fluorphenyl)-1-naphthalenyl]-phenyl}-2-propensäure,
- 3-{4-[3,4-Dihydro-6-methoxy-2-(4-fluorphenyl)-1-naphthalenyl]-phenyl}-2-propensäure,
- N,N-Diethyl-3-{4-[3,4-dihydro-6-methoxy-2-(4-fluorphenyl)-1-naphthalenyl]-phenyl}-2-propenamid,
- N,N-Diethyl-3-{4-[3,4-dihydro-6-hydroxy-2-(4-fluorphenyl)-1-naphthalenyl]-phenyl}-2-propenamid,
- (+,-) cis-3-{4-[6-Methoxy-2-(4-fluorphenyl)-1,2,3,4-tetrahydro-1-naphthalenyl]-phenyl}-2-propensäure-ethylester,
- (+,-) cis-3-{4-[6-Hydroxy-2-(4-fluorphenyl)-1,2,3,4-tetrahydro-1-naphthalenyl]-phenyl}-2-propensäure-ethylester,
- (+,-) cis-3-{4-[6-Hydroxy-2-(4-fluorphenyl)-1,2,3,4-tetrahydro-1-naphthalenyl]-phenyl}-2-propensäure,
- (+,-) cis-3-{4-[6-Methoxy-2-(4-fluorphenyl)-1,2,3,4-tetrahydro-1-naphthalenyl]-phenyl}-2-propensäure,
- (+,-) cis-N,N-Diethyl-3-{4-[6-methoxy-2-(4-fluorphenyl)-1,2,3,4-tetrahydro-1-naphthalenyl]-phenyl}-2-propenamid,
- (+,-) cis-N,N-Diethyl-3-{4-[6-hydroxy-2-(4-fluorphenyl)-1,2,3,4-tetrahydro-1-naphthalenyl]-phenyl}-2-propenamid,
- 3-[4-(3,4-Dihydro-6-methoxy-2-phenyl-1-naphthalenyl)-phenyl]-2-propensäure-ethylester,
- 3-[4-(3,4-Dihydro-6-hydroxy-2-phenyl-1-naphthalenyl)-phenyl]-2-propensäure-ethylester,
- (+,-) cis-3-[4-(6-Methoxy-2-phenyl)-1,2,3,4-tetrahydro-1-naphthalenyl)-phenyl]-2-propensäure-ethylester,
- (+,-) cis-3-[4-(6-Hydroxy-2-phenyl-1,2,3,4-tetrahydro-1-naphthalenyl)-phenyl]-2-propensäure-ethylester,
- (+,-) cis-N,N-Diethyl-3-[4-(6-methoxy-2-phenyl-1,2,3,4-tetrahydro-1-naphthalenyl)-phenyl]-2-propenamid,

wobei diese Verbindungen der Formel (I) in allen ihren möglichen stereoisomeren Formen, isoliert oder in Mischung, vorliegen können, sowie die Additionssalze mit Säuren und Basen.

**5.** Verfahren zur Herstellung der Verbindungen der Formel (I) nach Anspruch 1, **dadurch gekennzeichnet, daß** man eine Verbindung der Formel (II)

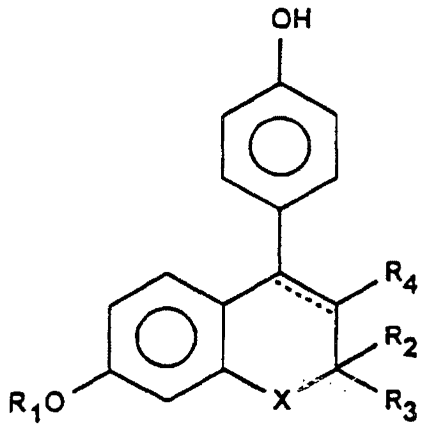

(II)

in der $R_1$, $R_2$, $R_3$, $R_4$ und X wie in Anspruch 1 definiert sind, der Einwirkung eines Mittels unterzieht, das vom Phenol aktiviert wird, und anschließend der Einwirkung einer Verbindung der Formel (III)

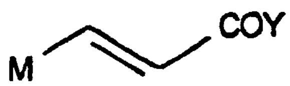

(III)

in der M ein Organometall-Derivat, vorzugsweise ein Organozinn-Derivat darstellt und Y wie in Anspruch 1 definiert ist, um die Verbindung der Formel (I) zu erhalten, die man gegebenenfalls, wenn erwünscht oder wenn erforderlich, bei einer oder mehreren der nachfolgenden Reaktionen in geeigneter Reihenfolge einsetzt:

- Alkylierung/Dealkylierung in Position 6,
- Acylierung/Spaltung von Acyloxy in Position 6,
- Veresterung/Verseifung der Säure oder des Esters, den COY darstellt,
- Amidierung der Säure oder des Esters, den COY darstellt,
- Salzbildung,
- Trennung der Diastereoisomeren, Auflösung und/oder Umwandlung in ein anderes Isomer.

**6.** Verfahren zur Herstellung der Verbindungen der Formel (I) nach Anspruch 1, worin die punktierte Linie eine Doppelbindung darstellt, **dadurch gekennzeichnet, daß** man eine Verbindung der Formel (IV)

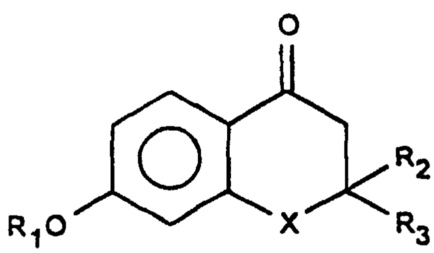

(IV)

in der $R_1$, $R_2$, $R_3$ und X wie in Anspruch 1 definiert sind, der Einwirkung einer organometallischen Magnesium- oder Lithium-Verbindung, die abgeleitet ist vom geschützten 4-Halogen-benzaldehyd, vorzugsweise in Anwesenheit von Ceriumchlorid unterzieht, um nach saurer Behandlung die Verbindung der Formel (V)

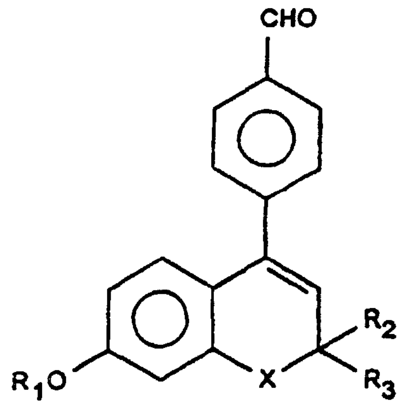

(V)

zu erhalten, die man der Einwirkung eines Reaktanden zur Halogenierung unterzieht, um eine Verbindung der Formel (VI)

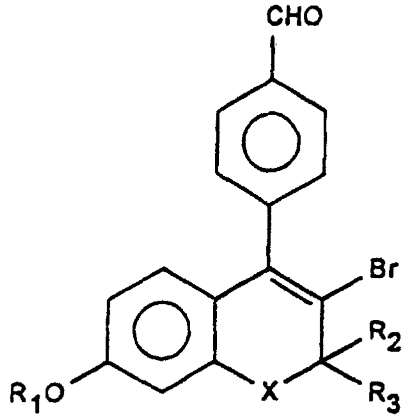

(VI)

zu erhalten, die man der Einwirkung eines Reaktanden zur Arylierung unterzieht, um eine Verbindung der Formel (VII)

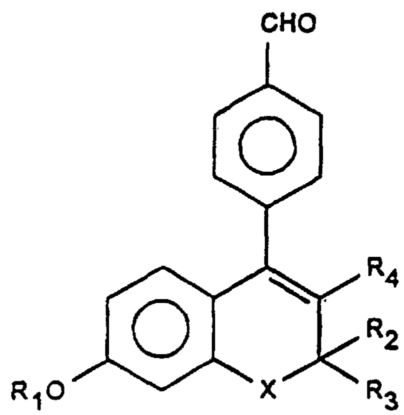

(VII)

zu erhalten, die man der Einwirkung eines Wittig-Reagens der Formel

ØP=CH-COY

unterwirft, in der Y wie in Anspruch 1 definiert ist, um die Verbindung der Formel (I) zu erhalten, die man gegebenenfalls, wenn erwünscht und wenn erforderlich, bei einer oder mehreren der nachfolgenden Reaktionen in geeigneter Reihenfolge einsetzt:

- Alkylierung/Dealkylierung in Position 6,
- Acylierung/Spaltung von Acyloxy in Position 6,
- Veresterung/Verseifung der Säure oder des Esters, den COY darstellt,
- Amidierung der Säure oder des Esters, den COY darstellt,
- Salzbildung,
- Trennung der Diastereoisomeren, Auflösung und/oder Umwandlung in ein anderes Isomer.

**7.** Verbindungen der Formel (I) wie in irgendeinem der Ansprüche 1 bis 3 definiert als Arzneimittel.

**8.** Verbindungen wie in Anspruch 4 definiert als Arzneimittel.

**9.** Pharmazeutische Zusammensetzungen, umfassend mindestens ein Arzneimittel wie in Anspruch 7 oder 8 definiert, sowie einen oder mehrere pharmazeutisch akzeptable Füllstoffe.

**10.** Verbindungen der Formeln (V), (VI) oder (VII) wie in Anspruch 6 definiert.

## Claims

**1.** Compounds of general formula (I) :

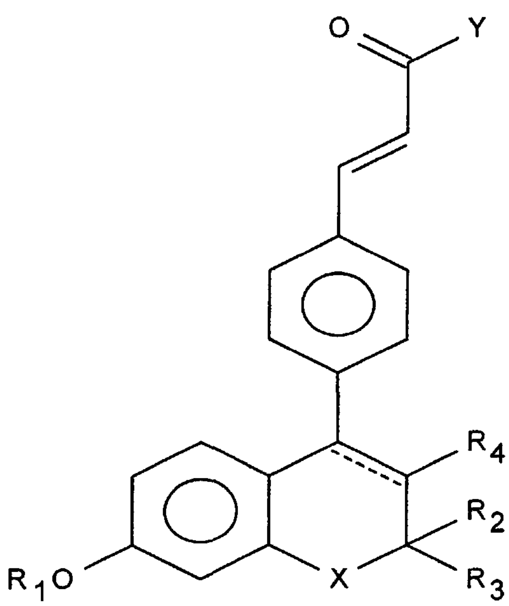

(I)

in which:

$R_1$ represents a hydrogen atom, an alkyl radical or an acyl radical,
$R_2$ and $R_3$, identical or different, represent a hydrogen atom, an alkyl radical, or together form a cycloalkyl containing from 3 to 7 carbon atoms,
$R_4$ represents an aryl or heteroaryl radical,
X represents O or $CH_2$,
Y represents a hydroxy, alkyloxy radical or an NRaRb group in which:

either Ra and Rb, identical or different, represent a hydrogen atom or an alkyl radical,
or Ra and Rb together with the nitrogen atom to which they are linked form a mono or polycyclic heterocycle of 3 to 15 members optionally containing an additional heteroatom chosen from oxygen, sulphur and nitrogen, said alkyl, alkyloxy and acyl containing from 1 to 6 carbon atoms, said alkyl, acyl, aryl and heterocycle are substituted or non substituted, the dotted line optionally represents a double bond, the compounds of formula (I) can be in all their possible stereoisomer forms, isolated or in mixtures, as well as the addition salts with acids or bases.

**2.** Compounds of formula (I) according to claim 1, in which:

$R_1$, $R_2$ and $R_3$ are hydrogen atoms, $R_4$ is a substituted or non substituted phenyl and Y represents a hydroxy, alkyloxy radical containing from 1 to 6 carbon atoms or NRaRb, Ra and Rb each representing an alkyl radical containing from 1 to 6 carbon atoms.

**3.** Compounds of formula (I) according to claim 1 or 2, in which the $R_4$ and Ph-CH=CH-COY groups are in cis position.

**4.** The following compounds of formula (I) according to claim 1:

- 3-[4-(3,4-dihydro-6-hydroxy-2-phenyl-1-naphthalenyl) phenyl]-2-propenoic acid,
- (+,-) cis 3-[4-(6-hydroxy-2-phenyl-1,2,3,4-tetrahydro-1-naphthalenyl)phenyl]-2-propenoic acid,
- (+,-) cis N,N-diethyl-3-[4-(6-hydroxy-2-phenyl-1,2,3,4-tetrahydro-1-naphthalenyl)phenyl]-2-propenamide,
- ethyl 3-[4-(3,4-dihydro-6-methoxy-2-(4-methoxyphenyl)-naphthalenyl) phenyl] 2-propenoate,
- ethyl 3-[4-(3,4-dihydro-6-hydroxy-2-(4-hydroxyphenyl) 1-naphthalenyl) phenyl] 2-propenoate,
- 3-[4-(3,4-dihydro-6-hydroxy-2-(4-hydroxyphenyl)-naphthalenyl) phenyl] 2-propenoic acid,
- 3-[4-(6-methoxy-2-(4-methoxyphenyl) 3,4-dihydro 1-naphthalenyl) phenyl] 2-propenoic acid,
- N,N-diethyl 3-[4-[3,4-dihydro-6-methoxy-2-(4-methoxyphenyl) 1-naphthalenyl] phenyl] 2-propenamide,
- N,N-diethyl 3-[4-[3,4-dihydro-6-hydroxy-2-(4-hydroxyphenyl) 1-naphthalenyl] phenyl] 2-propenamide,
- (+,-) cis ethyl 3-[4-[6-methoxy-2-(4-methoxyphenyl) 1,2,3,4-tetrahydro 1-naphthalenyl) phenyl] 2-propenoate,
- (+,-) cis ethyl 3-[4-[6-hydroxy-2-(4-hydroxyphenyl) 1,2,3,4-tetrahydro 1-naphthalenyl] phenyl] 2-propenoate,
- (+,-) cis 3-[4-[3,4-[6-methoxy-2-(4-methoxyphenyl)] 1,2,3,4-tetrahydro-1-naphthalenyl] phenyl] 2-propenoic acid,
- (+,-) cis 3-[4-[6-hydroxy-2-(4-hydroxyphenyl) 1,2,3,4-tetrahydro 1-naphthalenyl] phenyl] 2- propenoic acid,
- (+,-) cis N,N-diethyl-3-[4-[6-methoxy-2-(4-methoxyphenyl) 1,2,3,4-tetrahydro 1-naphthalenyl] phenyl] 2-propenamide,
- (+,-) cis N,N-diethyl-3-[4-[6-hydroxy-2-(4-hydroxyphenyl) 1,2,3,4-tetrahydro 1-naphthalenyl] phenyl] 2-propenamide,
- ethyl 3-[4-[3,4-dihydro-6-methoxy-2-(4-fluorophenyl) 1-naphthalenyl) phenyl] 2-propenoate,
- ethyl 3-[4-[3,4-dihydro-6-hydroxy-2-(4-fluorophenyl) 1-naphthalenyl) phenyl] 2-propenoate,
- 3-[4-[3,4-dihydro-6-hydroxy-2-(4-fluorophenyl) 1-naphthalenyl) phenyl] 2-propenoic acid,
- 3-[4-[6-methoxy-2-(4-fluorophenyl) 3,4-dihydro 1-naphthalenyl] phenyl] 2-propenoic acid,
- N,N-diethyl 3-[4-[3,4-dihydro-6-methoxy-2-(4-fluorophenyl) 1-naphthalenyl] phenyl] 2-propenamide,
- N,N-diethyl 3-[4-[3,4-dihydro-6-hydroxy-2-(4-fluorophenyl) 1-naphthalenyl] phenyl] 2-propenamide,
- (+,-) cis ethyl 3-[4-[6-methoxy-2-(4-fluorophenyl) 1,2,3,4-tetrahydro 1-naphthalenyl] phenyl] 2-propenoate,
- (+,-) cis ethyl 3-[4-[6-hydroxy-2-(4-fluorophenyl) 1,2,3,4-tetrahydro 1-naphthalenyl] phenyl] 2-propenoate,
- (+,-) cis 3-[4-[2-(4-fluorophenyl) 6-hydroxy 1,2,3,4-tetrahydro 1-naphthalenyl] phenyl] 2-propenoic acid,
- (+,-) cis 3-[4-[2-(4-fluorophenyl) 6-methoxy 1,2,3,4-tetrahydro 1-naphthalenyl] phenyl] 2-propenoic acid,
- (+,-) cis N,N-diethyl 3-[4-[6-methoxy-2-(4-fluorophenyl) 1,2,3,4-tetrahydro 1-naphthalenyl] phenyl] 2-propenamide,
- (+,-) cis N,N-diethyl 3-[4-[6-hydroxy-2-(4-fluorophenyl) 1,2,3,4-tetrahydro 1-naphthalenyl] phenyl] 2-propenamide,
- ethyl 3-[4-(3,4-dihydro-6-methoxy-2-phenyl-1-naphthalenyl) phenyl] 2-propenoate,
- ethyl 3-[4-(3,4-dihydro-6-hydroxy-2-phenyl-1-naphthalenyl) phenyl] 2-propenoate,
- (+,-) cis ethyl 3-[4-(6-methoxy-2-phenyl) 1,2,3,4-tetrahydro-1-naphthalenyl) phenyl] 2-propenoate,
- (+,-) cis ethyl 3-[4-(6-hydroxy-2-phenyl-1,2,3,4-tetrahydro-1-naphthalenyl) phenyl] 2-propenoate,
- (+,-) cis N,N-diethyl 3-[4-(6-methoxy-2-phenyl-1,2,3,4-tetrahydro 1-naphthalenyl) phenyl] 2-propenamide,

these compounds of formula (I) can be in all their possible isomer forms, isolated or in a mixture as well as the addition salts with acids and bases.

**5.** Process for the preparation of the compounds of formula (I) according to claim 1, **characterized in that** a compound of formula (II):

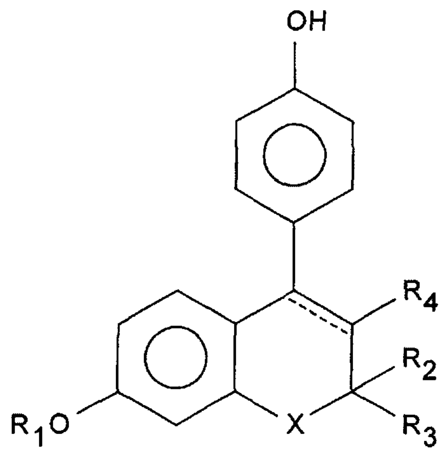

(II)

in which $R_1$, $R_2$, $R_3$, $R_4$ and X are as defined in claim 1, is subjected to the action of a phenol activating agent, then to the action of a compound of formula:

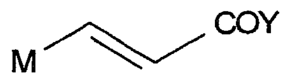

(III)

in which M is an organometallic derivative, preferably an organostannic, and Y is as defined in claim 1, in order to obtain the compound of formula (I) which if appropriate, if required or if necessary and in an appropriate order is used in one or more of the following reactions:

- alkylation/dealkylation in position 6,
- acylation/cleavage of the acyloxy in position 6,
- esterification/saponification of the acid or of the ester represented by COY,
- amidation of the acid or of the ester represented by COY,
- salification
- separation of the diastereoisomers, resolution and/or conversion in a other isomer.

**6.** Process for the preparation of the compounds of formula (I) according to claim 1, in which the dotted line represents a double bond, **characterized in that** a compound of formula (IV):

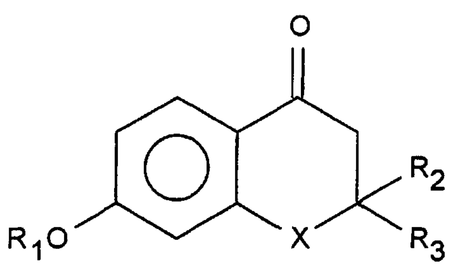

(IV)

$R_1$, $R_2$, $R_3$ and X being as defined in claim 1, is subjected to the action of a magnesium or lithium organo-metallic derivative of protected 4-halogeno benzaldehyde, preferably in the presence of cerium chloride, in order to obtain, after acid treatment, the compound of formula (V):

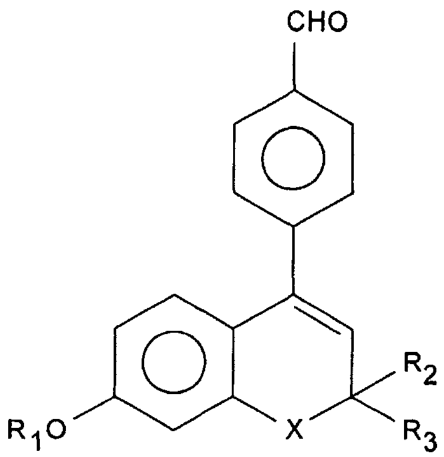

(V)

which compound of formula (V) is subjected to the action of a halogenation reagent in order to obtain a compound of formula (VI):

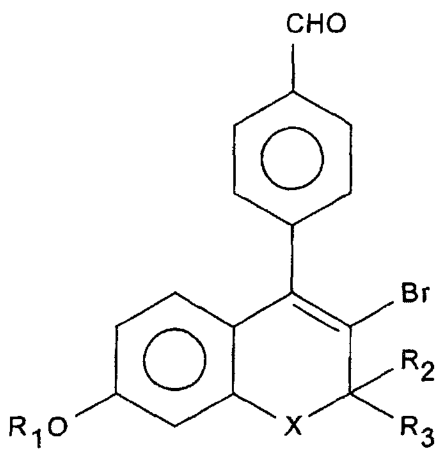

(VI)

which compound of formula (VI) is subjected to the action of an arylation reagent, in order to obtain a compound of formula (VII):

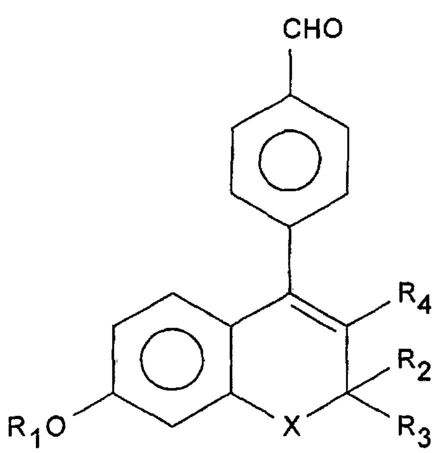

(VII)

which compound of formula (VII) is subjected to the action of a Wittig reagent of formula:

$\varphi_3P=CH-COY$

in which Y is as defined in claim 1, in order to obtain the compound of formula (I) which, if appropriate, if required and if necessary and in a appropriate order is used in one or more of the following reactions:

- alkylation/dealkylation in position 6,
- acylation/cleavage of the acyloxy in position 6,
- esterification/saponification of the acid or of the ester represented by COY,
- amidation of the acid or of the ester represented by COY,
- salification
- separation of the diastereoisomers, resolution and/or conversion to another isomer.

**7.** Compounds of formula (I) as defined in any one of claims 1 to 3, as medicaments.

**8.** Compounds as defined in claim 4, as medicaments.

**9.** Pharmaceutical compositions containing at least one medicament as defined in claim 7 or 8, as well as one or more pharmaceutically acceptable excipients.

**10.** Compounds of formulae (V), (VI) or (VII) as defined in claim 6.